# EUROPEAN PATENT APPLICATION

(11) **EP 4 375 382 A2**
(43) Date of publication of application: **29.05.2024**
(21) Application number: 24164850.0
(22) Date of filing: 12.08.2020
(51) Int. Cl.: C12R 1/465

(54) **BACTERIAL STRAINS HAVING FUNGICIDAL ACTIVITY, COMPOSITIONS COMPRISING SAME AND USE THEREOF**

(30) Priority: 14.08.2019 US 201962886359 P
(62) Divisional of application: 20852739.0
(71) Applicant: Lavie Bio Ltd., 7612002 Rehovot (IL)
(72) Inventor: VITERBO FAINZILBER, Ada, 7642414 Rehovot (IL); BERCOVITZ, Amir, 4333720 Raanana (IL); KUZNETS, Galit, 6794210 Tel Aviv (IL); ETZIONI, Adi, 9980300 Kibbutz Tzora (IL); MOVTCHAN, Anna, 7868866 Ashkelon (IL); KARCHI, Hagai, 7683400 Sitriya (IL); IONESCU, Michael, 7682327 Mazkeret Batya (IL)
(74) Representative: J A Kemp LLP

(57) **Abstract**

The present invention relates to the field of biocontrol of plant pathogenic fungi, particularly to bacterial strains effective in treating and/or preventing plant diseases associated with phytopathohenic fungi and/or oomycetes, preparations, lysates and extracts thereof and compositions comprising same, and methods of use thereof.

## Description

### FIELD OF THE INVENTION

The present invention relates to the field of biocontrol of plant pathogenic fungi, particularly to bacterial strains effective in treating and/or preventing plant diseases associated with phytopathogenic fungi and/or oomycetes; lysates and extracts thereof, compositions comprising same and use thereof.

### BACKGROUND OF THE INVENTION

During all developmental stages, plants are exposed to an extremely wide range of biotic and abiotic stress conditions leading to plant diseases. In the production of crop plants, damages caused by biotic stresses, particularly by pathogenic agents, which may be further enhanced under conditions of abiotic stress, pose a major problem and significantly affect the crop yield and profitability.

Many plant diseases are caused by plant pathogenic fungi and oomycetes, and yearly damages in yield to both monocotyledonous and dicotyledonous crop plants amount to billions of US$ in the U.S. alone. For example, *Fusarium* seedling blight symptoms are caused by several *Fusarium* species, particularly *Fusarium oxysporum* and *Fusarium graminearum,* which cause the disease in tomato and corn seedlings, respectively. *Botrytis cinerea* causes Gray Mold disease in tomato fruits, grapes and many other fruits and berries.

Downy Mildew is one of the most serious grapevine (*Vitis vinifera*) diseases in the world. It is caused by the biotrophic oomycete *Plasmopara viticola,* which can attack all green parts of the grapevine. No effective biological solutions are available nowadays against this disease, and chemical treatments afford only partial protection. *Erysiphe necator* causes powdery mildew disease, which also causes vast damage in grapevine growth. Unlike many other fungal diseases, powdery mildew thrives in warm, dry climates, though it does require fairly high relative humidity. The powdery mildew fungus can infect all green tissues of the vine, and can result in reduced vine growth, yield, fruit quality, and winter hardiness.

Fungal and oomycete pathogens are typically controlled by the use of synthetic chemicals (e.g., fungicides). However, although may be effective, synthetic chemicals increase agricultural production costs and, moreover, are typically toxic to animals and humans and have harmful effects on the environment. Additionally, pathogen resistance to such chemicals is rising as a result of overuse. In some countries, certain fungicides or anti-oomycete chemicals have been restricted or banned for these and other reasons. Therefore, there is a growing interest in developing control methods and compositions that do not rely on toxic synthetic chemical fungicides or anti-oomycete compounds, or that reduce the use of such chemicals.

Biocontrol agents are typically microorganisms, such as bacteria or one or more products thereof that are applied to a plant or a part thereof, or to the plant habitat, to control a pathogen. Biocontrol agents are good candidates to replace toxic fungicides/ anti-oomycete compounds. For example, International (PCT) Patent Application Publication No. WO 2014/173906 discloses a novel bacterial strain, *Lysobacter capsici,* and uses thereof for plant protection, particularly for protecting plants from pathogenic fungi and/or oomycetes. The invention also relates to the combined use of compositions comprising copper (such as copper-containing plant protection products) and *Lysobacter capsici* bacteria in treating such pathogens.

International (PCT) Patent Application Publication No. WO 2016/156164 discloses strain CECT8836 of *Bacillus amyloliquefaciens* and mutants thereof, and the use of said strain, extracts thereof and compositions comprising same as a pesticide in controlling plant diseases caused by fungi and bacteria.

However, there is a limited number of commercially available biocontrol agents. Furthermore, most known biocontrol agents are limited to control of single phytopathogens in their known effect and/or their practical use. For these and other reasons, there remains a need for compositions containing biocontrol agents for the control of a broad spectrum of pathogenic fungi and/or oomycetes.

### SUMMARY OF THE INVENTION

The present invention answers the need for safe biocontrol agents that can protect plants, particularly crop plants, from a broad spectrum of diseases caused by pathogenic fungi and/or oomycetes. The present invention provides bacterial strains showing unexpectedly effective activity in combating a wide range of phytopathogenic fungi and/or oomycetes. The present invention further provides preparations of the bacterial strains, lysates, extracts, compositions comprising same and uses thereof. In certain aspects the present invention provides methods for conferring or enhancing the resistance of plants towards a broad spectrum of pathogenic fungi and/or oomycetes, comprising contacting the plants, or the plants' immediate surroundings, with the bacterial strain or a combination of bacterial strains.

According to one aspect, the present invention provides an isolated bacterial strain or a functional homologue thereof, wherein the isolated bacterial strain is selected from the group consisting of:
(1) strain LAV49762, the strain being selected from the group consisting of
   a. a strain deposited under Accession Number 43430 at NCIMB;
   b. a strain comprising a 16S-rRNA sequence comprising the nucleic acid sequence set forth in SEQ ID NO: 10;
   c. a strain comprising at least one genomic marker comprising the nucleic acid sequence set forth in any one of SEQ ID NOs:53-57;
   and any combination thereof;
(2) strain LAV34085, the strain being selected from the group consisting of
   a. a strain deposited under Accession Number 43434 at NCIMB;
   b. a strain comprising a 16S-rRNA sequence comprising the nucleic acid sequence set forth in SEQ ID NO:1;
   c. a strain comprising at least one genomic marker comprising the nucleic acid sequence set forth in any one of SEQ ID NOs:21-25;
   and any combination thereof;
(3) strain LAV43122, the strain being selected from the group consisting of
   a. a strain deposited under Accession Number 43431 at NCIMB;
   b. a strain comprising at least one 16S-rRNA sequence comprising the nucleic acid sequence selected from the group consisting of SEQ ID NO:2 and SEQ ID NO:3;
   c. a strain comprising at least one genomic marker comprising the nucleic acid sequence set forth in any one of SEQ ID NOs:26-27;
   and any combination thereof;
(4) strain LAV43723, the strain being selected from the group consisting of
   a. a strain deposited under Accession Number 43436 at NCIMB;
   b. a strain comprising a 16S-rRNA sequence comprising the nucleic acid sequence set forth in SEQ ID NO:4;
   c. a strain comprising at least one genomic marker comprising the nucleic acid sequence set forth in any one of SEQ ID NOs:28-32;
   and any combination thereof;
(5) strain LAV46348, the strain being selected from the group consisting of
   a. a strain deposited under Accession Number 43435 at NCIMB;
   b. a strain comprising a 16S-rRNA sequence comprising the nucleic acid sequence set forth in SEQ ID NO:5;
   c. a strain comprising at least one genomic marker comprising the nucleic acid sequence set forth in any one of SEQ ID NOs:33-37;
   and any combination thereof;
(6) strain LAV49623, the strain being selected from the group consisting of
   a. a strain deposited under Accession Number 43429 at NCIMB;
   b. a strain comprising a 16S-rRNA sequence comprising the nucleic acid sequence set forth in SEQ ID NO:7;
   c. a strain comprising at least one genomic marker comprising the nucleic acid sequence set forth in any one of SEQ ID NOs:43-47;
   and any combination thereof;
(7) strain LAV49648, the strain being selected from the group consisting of
   a. a strain deposited under Accession Number 43432 at NCIMB;
   b. a strain comprising at least one 16S-rRNA sequence comprising the nucleic acid sequence selected from the group consisting of SEQ ID NO:8 and SEQ ID NO:9;
   c. a strain comprising at least one genomic nucleic acid marker comprising the nucleic acid sequence set forth in any one of SEQ ID NOs:48-52;
   and any combination thereof;
(8) strain LAV54823, the strain being selected from the group consisting of
   a. a strain deposited under Accession Number 43433 at NCIMB; and
   b. a strain comprising a 16S-rRNA sequence comprising the nucleic acid sequence set forth in SEQ ID NO:11;
   and a combination thereof;
(9) strain LAV60069, the strain being selected from the group consisting of
   a. a strain deposited under Accession Number 43606 at NCIMB;
   b. a strain comprising a 16S-rRNA sequence comprising the nucleic acid sequence set forth in SEQ ID NO:16;
   c. a strain comprising at least one genomic marker comprising the nucleic acid sequence set forth in any one of SEQ ID NOs:67-71;
   and any combination thereof;
(10) strain LAV61190, the strain being selected from the group consisting of
   a. a strain deposited under Accession Number 43607 at NCIMB;
   b. a strain comprising a 16S-rRNA sequence comprising the nucleic acid sequence set forth in SEQ ID NO:20;
   and a combination thereof;
(11) strain LAV60070, the strain being selected from the group consisting of
   a. a strain deposited under Accession Number 43608 at NCIMB;
   b. a strain comprising at least one 16S-rRNA sequence comprising the nucleic acid sequence selected from the group consisting of SEQ ID NO: 17 and SEQ ID NO:18;
   and a combination thereof;
(12) strain LAV60067, the strain being selected from the group consisting of
   a. a strain deposited under Accession Number 43609 at NCIMB;
   b. a strain comprising a 16S-rRNA sequence comprising the nucleic acid sequence set forth in SEQ ID NO:15;
   c. a strain comprising at least one genomic marker comprising the nucleic acid sequence set forth in any one of SEQ ID NOs:62-66;
   and any combination thereof;
(13) strain LAV59924, the strain being selected from the group consisting of
   a. a strain deposited under Accession Number 43610 at NCIMB;
   b. a strain comprising a 16S-rRNA sequence comprising the nucleic acid sequence set forth in SEQ ID NO: 12;
   and a combination thereof;
(14) strain LAV48853, the strain being selected from the group consisting of
   a. a strain deposited under Accession Number 43611 at NCIMB;
   b. a strain comprising a 16S-rRNA sequence comprising the nucleic acid sequence set forth in SEQ ID NO:6;
   c. a strain comprising at least one genomic marker comprising the nucleic acid sequence set forth in any one of SEQ ID NOs:38-42;
   and any combination thereof;
(15) strain LAV60063, the strain being selected from the group consisting of
   a. a strain deposited under Accession Number 43612 at NCIMB;
   b. a strain comprising a 16S-rRNA sequence comprising the nucleic acid sequence set forth in SEQ ID NO:13;
   c. a strain comprising at least one genomic marker comprising the nucleic acid sequence set forth in any one of SEQ ID NOs:58-59;
   and any combination thereof;
(16) strain LAV60064, the strain being selected from the group consisting of
   a. a strain deposited under Accession Number 43613 at NCIMB;
   b. a strain comprising a 16S-rRNA sequence comprising the nucleic acid sequence set forth in SEQ ID NO:14;
   c. a strain comprising at least one genomic marker comprising the nucleic acid sequence set forth in any one of SEQ ID NOs:60-61;
   and any combination thereof; and
(17) strain LAV60072, the strain being selected from the group consisting of
   a. a strain deposited under Accession Number 43614 at NCIMB;
   b. a strain comprising a 16S-rRNA sequence comprising the nucleic acid sequence set forth in SEQ ID NO:19;
   c. a strain comprising at least one genomic marker comprising the nucleic acid sequence set forth in SEQ ID NOs:72;
   and any combination thereof.

Each possibility represents a separate embodiment of the present invention.

According to certain embodiments, strain LAV49762, strain LAV46348, strain LAV43122, strain LAV48853, strain LAV60063, strain LAV60064, and strain LAV60072 are of the genus *Pseudomonas.*

According to certain embodiments, strain LAV34085 is of the genus *Serratia.* According to certain exemplary embodiments, strain LAV34085 is of the species *Serratia plymutica.*

According to certain embodiments, strain LAV43723 is of the genus *Streptomyces.*

According to certain embodiments, strain LAV49623 and strain LAV59924 are of the genus *Pantoea.* According to certain embodiments, strain LAV49648 and strain LAV60070 are of the genus *Enterobacter.*

According to certain embodiments, strain LAV54823 is of the genus *Erwinia.*

According to certain embodiments, strain LAV60069 and strain LAV61190 are of the genus *Bacillus.*

According to certain embodiments, strain LAV60067 is of the genus *Gluconobacter.*

According to certain embodiments, the functional homolog of bacterial strain LAV49762 comprises a 16S-rRNA sequence at least 97% identical to SEQ ID NO: 10; at least one genomic marker having at least 95% local identity to a nucleic acid sequence set forth in any one of SEQ ID NOs:53-57 over 90% coverage; or a combination thereof.

According to certain embodiments, the functional homolog of bacterial strain LAV34085 comprises a 16S-rRNA sequence at least 97% identical to SEQ ID NO:1; at least one genomic marker having at least 95% local identity to a nucleic acid sequence set forth in any one of SEQ ID NOs:21-25 over 90% coverage; or a combination thereof.

According to certain embodiments, the functional homolog of bacterial strain LAV43122 comprises a 16S-rRNA sequence having the nucleic acid sequence set forth in any one of SEQ ID NO:2 and SEQ ID NO:3 and a genomic marker having at least 95% local identity to a nucleic acid sequence set forth in any one of SEQ ID NOs:26-27 over 90% coverage; or a combination thereof.

According to certain embodiments, the functional homolog of bacterial strain LAV43723 comprises a 16S-rRNA sequence at least 97% identical to SEQ ID NO:4; at least one genomic marker having at least 95% local identity to a nucleic acid sequence set forth in any one of SEQ ID NOs:28-32 over 90% coverage; or a combination thereof.

According to certain embodiments, the functional homolog of bacterial strain LAV46348 comprises a 16S-rRNA sequence at least 98% identical to SEQ ID NO:5; at least one genomic marker having at least 95% local identity to a nucleic acid sequence set forth in any one of SEQ ID NOs:33-37 over 90% coverage; or a combination thereof.

According to certain embodiments, the functional homolog of bacterial strain LAV49623 comprises a 16S-rRNA sequence at least 97% identical to SEQ ID NO:7; at least one genomic marker having at least 95% local identity to a nucleic acid sequence set forth in any one of SEQ ID NOs:43-47 over 90% coverage; or a combination thereof.

According to certain embodiments, the functional homolog of bacterial strain LAV49648 comprises a 16S-rRNA sequence having the nucleic acid sequence set forth in any one of SEQ ID NO:8 and SEQ ID NO:9 and a genomic marker having at least 95% local identity to a nucleic acid sequence set forth in any one of SEQ ID NOs:48-52 over 90% coverage; or a combination thereof.

According to certain embodiments, the functional homolog of bacterial strain LAV60069 comprises a 16S-rRNA sequence at least 97% identical to SEQ ID NO:16; at least one genomic marker having at least 95% local identity to a nucleic acid sequence set forth in any one of SEQ ID NOs:67-71 over 90% coverage; or a combination thereof.

According to certain embodiments, the functional homolog of bacterial strain LAV61190 comprises a 16S-rRNA sequence at least 97% identical to SEQ ID NO:20.

According to certain embodiments, the functional homolog of bacterial strain LAV60067 comprises a 16S-rRNA sequence at least 97% identical to SEQ ID NO:15; at least one genomic marker having at least 95% local identity to a nucleic acid sequence set forth in any one of SEQ ID NOs:62-66 over 90% coverage; or a combination thereof.

According to certain embodiments, the functional homolog of bacterial strain LAV59924 comprises a 16S-rRNA sequence at least 97% identical to SEQ ID NO:12.

According to certain embodiments, the functional homolog of bacterial strain LAV48853 comprises a 16S-rRNA sequence at least 99% identical to SEQ ID NO:6; at least one genomic marker having at least 95% local identity to a nucleic acid sequence set forth in any one of SEQ ID NOs:38-42 over 90% coverage; or a combination thereof.

According to certain embodiments, the functional homolog of bacterial strain LAV60063 comprises a 16S-rRNA sequence at least 99% identical to SEQ ID NO:13; at least one genomic marker having at least 95% local identity to a nucleic acid sequence set forth in any one of SEQ ID NOs:58-59 over 90% coverage; or a combination thereof.

According to certain embodiments, the functional homolog of bacterial strain LAV60064 comprises a 16S-rRNA sequence at least 97% identical to SEQ ID NO:14; at least one genomic marker having at least 95% local identity to a nucleic acid sequence set forth in any one of SEQ ID NOs:60-61 over 90% coverage; or a combination thereof.

According to certain embodiments, the functional homolog of bacterial strain LAV60072 comprises a 16S-rRNA sequence at least 97% identical to SEQ ID NO:19; a genomic marker having at least 95% local identity to the nucleic acid sequence set forth in SEQ ID NO:72 over 90% coverage; or a combination thereof.

It is to be explicitly understood that the present invention encompasses a bacterium of the bacterial strains or the functional homolog strains thereof as well as a bacterium derivable from bacterial strains or the functional homolog strains thereof.

According to certain embodiments, the bacterial strains, functional homologous bacterial strains and bacterium derived therefrom are characterized by a capability to inhibit the growth and/or the development and/or the activity of at least one plant pathogenic fungus and/or oomycete.

According to another aspect, the present invention provides a bacterial preparation comprising a plurality of bacteria of at least one bacterial strain selected from the group consisting of LAV49762, LAV34085, LAV43122, LAV43723, LAV46348, LAV49623, LAV49648, LAV54823, LAV60069, LAV61190, LAV60070, LAV60067, LAV59924, LAV48853, LAV60063, LAV60064, LAV60072 and functional homologs thereof, wherein the bacterial strain or functional homolog thereof is effective in protecting a plant from at least one pathogenic fungus and/or oomycete. Each possibility represents a separate embodiment of the present invention.

According to certain embodiments, the bacterial preparation comprises a culture medium. Culture media suitable for the growth of the various bacterial genera and species according to the teachings of the present invention are known to a person skilled in the Art.

According to certain embodiments, the bacterial strain or functional homolog thereof is present in the preparation at a concentration which exceeds that found in nature.

According to certain embodiments, the bacterial preparation comprises viable bacterial cells. According to other embodiments, the bacterial preparation comprises non-viable cells.

Additionally, the present invention now shows that an autoclaved culture comprising bacterial strains of the present invention fully preserved the anti-fungicidal effect of the strains. Without wishing to be bound by any specific theory or mechanism of action, the preserved anti-fungal activity may be attributed to compounds maintaining their activity after being released from the bacterial cells and/or compounds activated under such conditions.

According to certain embodiments, the bacterial preparation comprises viable bacterial cells. According to other embodiments, the bacterial preparation comprises non-viable cells. According to certain exemplary embodiments, the present invention provides a bacterial preparation subjected to a temperature of from about 100°C to about 130°C under pressure of at least 15 psi for at least 10min, at least 20min, or at least 30 min. According to certain exemplary embodiments the bacterial preparation has been autoclaved under conditions of 121°C for at least 30 min at 15 psi.

According to yet an additional aspect, the present invention provides a lysate of at least one bacterial strain of the invention or the functional homologs thereof. According to some embodiments, the lysate comprises a whole cell lysate of the bacterial cells. According to some embodiments, the lysate comprises soluble fraction of the bacterial cells.

According to yet an additional aspect, the present invention provides a cell extract of at least one bacterial strain of the invention or the functional homologs thereof.

According to certain embodiments, the lysate or extract is of a plurality of the bacterial cells. According to certain embodiments, the lysate or extract is of bacterial cells of the same bacterial species and/or strain. According to certain embodiments, the lysate or extract is of bacterial cells of different species and/or strains.

The bacterial strain and functional homologs thereof are as described hereinabove.

According to a further aspect, the present invention provides an agricultural composition comprising at least one bacterial strain selected from the group consisting of LAV49762, LAV34085, LAV43122, LAV43723, LAV46348, LAV49623, LAV49648, LAV54823, LAV60069, LAV61190, LAV60070, LAV60067, LAV59924, LAV48853, LAV60063, LAV60064, LAV60072, and functional homologs thereof, a preparation of same, a lysate thereof, or an extract thereof, further comprising an agriculturally acceptable diluent(s) or carrier(s). Each possibility represents a separate embodiment of the present invention.

According to yet an additional aspect, the present invention provides use of at least one bacterial strain selected from the group consisting of LAV49762, LAV34085, LAV43122, LAV43723, LAV46348, LAV49623, LAV49648, LAV54823, LAV60069, LAV61190, LAV60070, LAV60067, LAV59924, LAV48853, LAV60063, LAV60064, LAV60072, and functional homologs thereof, a preparation of same, or a lysate thereof for the production of an agricultural composition. Each possibility represents a separate embodiment of the present invention.

According to certain embodiments, the agricultural composition is a plant protection product effective in preventing or treating at least one plant disease caused by a pathogenic fungus and/or oomycete.

According to certain embodiments, the agricultural composition further comprises at least one additional active agent selected from the group consisting of a fertilizer, an acaricide, a bactericide, a fungicide, an insecticide, a microbicide, a nematicide, a pesticide, a plant growth regulator, a rodenticide, a nutrient and any combination thereof. Each possibility represents a separate embodiment of the present invention. According to certain embodiments, the at least one additional active agent is a synthetic agent.

The agricultural composition can be formulated in any form suitable for applying the composition to a plant or a part thereof as is known in the art. According to certain embodiments, the agricultural composition is formulated in a form selected from the group consisting of an emulsion, a colloid, a dust, a granule, a pellet, a powder, a spray and a solution. Each possibility represents a separate embodiment of the present invention.

According to certain embodiments, the formulation further comprises at least one of a stabilizer, a tackifier, a preservative, a carrier, a surfactant, and a combination thereof. Each possibility represents a separate embodiment of the present invention.

According to certain embodiments, the formulation is substantially stable for at least 180 days at a temperature range of from about 4°C to about 37°C. According to certain exemplary embodiments, the formulation is substantially stable at a temperature range of from about 20°C to 25°C for more than 180 days. According to certain additional exemplary embodiments, the formulation is substantially stable at a temperature range of from about 2-8°C, typically at 4°C, for at least 180 days.

According to some embodiments of the invention, the formulation is a liquid, solid, semi-solid, gel or powder.

According to certain embodiments, the carrier is a plant seed. According to these embodiments, the present invention comprises an agricultural composition comprising at least one plant seed and at least one bacterial strain selected from the group consisting of LAV49762, LAV34085, LAV43122, LAV43723, LAV46348, LAV49623, LAV49648, LAV54823, LAV60069, LAV61190, LAV60070, LAV60067, LAV59924, LAV48853, LAV60063, LAV60064, LAV60072, and functional homologs thereof, a preparation of same, a lysate thereof, or an extract thereof.

According to a further aspect, the present invention provides a method for enhancing and/or conferring resistance of a plant or a part thereof toward at least one disease caused by a phytopathogenic fungus and/or oomycete, comprising contacting the plant or part thereof with at least one bacterial strain selected from the group consisting of LAV60072, LAV34085, LAV43122, LAV43723, LAV46348, LAV49623, LAV49648, LAV49762, LAV54823, LAV60069, LAV61190, LAV60070, LAV60067, LAV59924, LAV48853, LAV60063, LAV60064, and functional homologs thereof, a preparation of same, a lysate thereof, an extract thereof or a composition comprising same. Each possibility represents a separate embodiment of the present invention.

According to certain embodiments, the plant is susceptible to the at least one disease caused by the phytopathogenic fungus and/or oomycete. According to some embodiments, the method further comprises identifying a plant to be susceptible to the at least one disease caused by the phytopathogenic fungus and/or oomycete before contacting said plant or a part thereof with the at least one bacterial strain, functional homolog thereof, preparation of same, lysate thereof or a composition comprising same.

According to yet a further aspect, the present invention provides a method for preventing or treating a plant disease caused by a phytopathogenic fungus and/or oomycete, comprising contacting a plant or a part thereof with at least one bacterial strain selected from the group consisting of LAV49762, LAV34085, LAV43122, LAV43723, LAV46348, LAV49623, LAV49648, LAV54823, LAV60069, LAV61190, LAV60070, LAV60067, LAV59924, LAV48853, LAV60063, LAV60064, LAV60072, and functional homologs thereof, a preparation of same, a lysate thereof, an extract thereof or a composition comprising same. Each possibility represents a separate embodiment of the present invention.

According to certain embodiments, the plant is affected by the disease caused by the phytopathogenic fungus and/or oomycete. According to some embodiments, the method further comprises identifying symptoms of the disease within the plant before contacting said plant or a part thereof with the at least one bacterial strain, functional homolog thereof, preparation of same, lysate thereof or a composition comprising same.

The bacterial strains are as described herein above. According to certain embodiments, the methods of the present invention comprise contacting the plant or a part thereof with at least one functional homolog of strains LAV49762, LAV34085, LAV43723, LAV46348, LAV49623, LAV60069, LAV61190, LAV60067, LAV59924, LAV48853, LAV60063, LAV60064, LAV60072, as described hereinabove, preparations of same, lysate thereof, extract thereof or a composition comprising same.

According to certain embodiments, the methods of the invention comprise contacting the plant or part thereof with a functional homolog of bacterial strain LAV43122, the functional homolog comprising a 16S-rRNA sequence at least 97% identical to a nucleic acid sequence selected from the group consisting of SEQ ID NO:2 and SEQ ID NO:3; a genomic marker having at least 95% local identity to a nucleic acid sequence set forth in any one of SEQ ID NOs:26-27 over 90% coverage; or a combination thereof, a preparation of same, lysate thereof or a composition comprising same.

According to certain embodiments, the methods of the invention comprise contacting the plant or part thereof with a functional homolog of bacterial strain LAV49648 the functional homolog comprising a 16S-rRNA sequence at least 97% identical to a nucleic acid sequence selected from the group consisting of SEQ ID NO:8 and SEQ ID NO:9; a genomic marker having at least 95% local identity to a nucleic acid sequence set forth in any one of SEQ ID NOs:48-52 over 90% coverage; or a combination thereof, a preparation of same, lysate thereof or a composition comprising same.

According to certain embodiments, the methods of the invention comprise contacting the plant or part thereof with a functional homolog of bacterial strain LAV54823, the functional homolog comprising a 16S-rRNA sequence at least 97% identical to a nucleic acid sequence set forth in SEQ ID NO:11, a preparation of same, lysate thereof or a composition comprising same.

According to certain embodiments, the methods of the invention comprise contacting the plant or part thereof with a functional homolog of bacterial strain LAV60070, the functional homolog comprising a 16S-rRNA sequence selected from the group consisting of a 16S-rRNA at least 97% identical to the nucleic acid sequence set forth in SEQ ID NO:17 and a 16S-rRNA having the nucleic acid sequence set forth in SEQ ID NO:18, a preparation of same, lysate thereof or a composition comprising same.

According to certain embodiments, the plant part is selected from the group consisting of a seed, a root, a shoot, a leaf, a branch, a flower, a fruit and any combination thereof. Each possibility represents a separate embodiment of the present invention.

Any method as is known in the art for contacting bacterial strains with a plant or part thereof can be used according to the teachings of the present invention. Typically, the plant or part thereof is contacted with a composition comprising the bacterial strains, functional homologs thereof or preparations of same. The plant can be contacted directly with the composition or the composition may be applied to the plant growth medium, which can be a solid or a liquid growth medium.

According to certain embodiments, the composition is formulated in a liquid form. According to these embodiments, the plant or part thereof may be contacted with the composition by a method selected from the group consisting of infiltration, immersion/dipping, incubation, spraying, and any combination thereof. Each possibility represents a separate embodiment of the present invention.

According to certain exemplary embodiments, the plant part is a seed. According to certain embodiments, the at least one bacterial strain according to the teachings of the invention is applied to the seed via seed coating.

According to certain additional or alternative exemplary embodiments, the plant part is a leaf. According to some embodiments, the at least one bacterial strain is applied to the leaf by spraying a bacterial preparation or a composition comprising same according to the teachings of the present invention.

According to certain exemplary embodiments, the bacterial strain is applied at a concentration range of from about 10⁵ CFU/ml to about 10⁸ CFU/ml of the bacterial preparation or the agricultural composition.

According to certain embodiments, the phytopathogenic fungus is of a genus selected from the group consisting of *Fusarium, Botrytis, Erysiphe, Aspergillus* and *Rhizopus.* Each possibility represents a separate embodiment of the present invention.

According to certain exemplary embodiments, the phytopathogenic fungus is of a genus selected from the group consisting of *Fusarium, Botrytis* and *Erysiphe.* Each possibility represents a separate embodiment of the present invention.

According to certain embodiments, the phytopathogenic fungus is selected from the group consisting of, *Fusarium graminearum, Fusarium oxysporum, Botrytis cinerea, Erysiphe necator, Fusarium verticillioides*, *Aspergillus niger,* and *Rhizopus stolonifera.* Each possibility represents a separate embodiment of the present invention.

According to certain exemplary embodiments, the phytopathogenic fungus is selected from the group consisting of *Fusarium graminearum, Fusarium oxysporum*, *Fusarium verticillioides*, *Botrytis cinerea* and *Erysiphe necator,*

According to certain embodiments, the oomycete is of a genus selected from the group consisting of *Plasmopara, Phytophthora, Pythium* and *Pseudoperonospora.* Each possibility represents a separate embodiment of the present invention.

According to certain exemplary embodiments, the oomycete is of a genus selected from the group consisting of *Plasmopara* and *Phytophthora.*

According to certain embodiments, the oomycete is selected from the group consisting of *Plasmopara viticola, Phytophthora capsica,* and *Pseudoperonospora cubensis.* Each possibility represents a separate embodiment of the present invention.

According to certain exemplary embodiments, the oomycete is selected from the group consisting of *Plasmopara viticola* and *Phytophthora capsica.*

According to certain exemplary embodiments, the pathogenic fungus is selected from the group consisting of *Fusarium graminearum, Fusarium verticilloides,* and a combination thereof, and the bacterial strain is selected from the group consisting of LAV43122, LAV46348, LAV49623, LAV60067, LAV61190, functional homologs thereof and any combination thereof. Each possibility represents a separate embodiment of the present invention.

According to certain exemplary embodiments, the pathogenic oomycete is *Phytophthora capsici* and the bacterial strain is selected from the group consisting of LAV43122, LAV48853, LAV59924, LAV60064, LAV60067, LAV60072, LAV61190, functional homologs thereof, and any combination thereof. Each possibility represents a separate embodiment of the present invention.

According to certain exemplary embodiments, the pathogenic oomycete is *Plasmopara viticola,* which causes Downy Mildew disease, and the bacterial strain is selected from the group consisting of LAV43723, LAV61190, functional homologs thereof and any combination thereof. Each possibility represents a separate embodiment of the present invention. According to certain exemplary embodiments, the plant contacted with the bacterial strain is a grapevine.

According to certain exemplary embodiments, the pathogenic fungus is *Erysiphe necator,* which causes Powdery Mildew disease, and the bacterial strain is selected from the group consisting of LAV49648, LAV43122, LAV54823, LAV48853, LAV60062, LAV60063, LAV60069, LAV60070, LAV60072, functional homologs thereof, and any combination thereof. Each possibility represents a separate embodiment of the present invention. According to certain exemplary embodiments, the plant contacted with the bacterial strain is a grapevine.

According to certain exemplary embodiments, the pathogenic fungus is *Botrytis cinerea,* which causes Gray Mold disease, and the bacterial strain is selected from the group consisting of LAV49762, LAV43122, LAV54823, LAV49648, LAV49623, LAV34085, LAV46348, LAV59924, LAV60063, LAV60064, LAV60067, LAV60069, LAV60070, LAV60072, LAV60096, functional homologs thereof and any combination thereof. Each possibility represents a separate embodiment of the present invention. According to certain exemplary embodiments, the plant contacted with the bacterial strain is a tomato plant. According to certain exemplary embodiments, the plant contacted with the bacterial strain is a grapevine.

According to certain exemplary embodiments, the pathogenic fungus is *Aspergillus niger,* which causes Black Mold disease, and the bacterial strain is selected from the group consisting of LAV49762, LAV54823, functional homologs thereof and a combination thereof. Each possibility represents a separate embodiment of the present invention. According to certain exemplary embodiments, the plant contacted with the bacterial strain is a grapevine.

According to certain exemplary embodiments, the pathogenic fungus is selected from the group consisting of *Fusarium oxysporum, Fusarium graminearum,* and a combination thereof, which cause *Fusarium* seedling blight symptoms, and the bacterial strain is selected from the group consisting of LAV43122, LAV46348, LAV49623, LAV60067, LAV61190, functional homologs thereof and any combination thereof. Each possibility represents a separate embodiment of the present invention. According to certain exemplary embodiments, the plant contacted with the bacterial strain is selected from the group consisting of a tomato plant and a corn plant.

It is to be understood that any combination of each of the aspects and the embodiments disclosed herein is explicitly encompassed within the disclosure of the present invention.

Other objects, features and advantages of the present invention will become clear from the following description.

### DETAILED DESCRIPTION OF THE INVENTION

The present invention provides isolated microbial strains, particularly bacterial strains, not hitherto described, wherein the bacterial strains are characterized by effective anti-fungal and/or anti-oomycete activity and therefore are useful as biocontrol agents for protecting plants from diseases caused by the fungi and/or oomycetes. The anti-fungal/oomycete activity encompasses inhibiting at least one of growth, development and phytopathogenic activity of at least one of the fungi/oomycetes.

### Definitions

The terms "comprise", "comprising", "includes", "including", "having" and their conjugates mean "including but not limited to".

The term "consisting of' means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the agricultural, chemical, pharmacological, biological, biochemical and medical arts.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

As used herein, the terms "sequence identity" or "identity" or grammatical equivalents, in the context of two nucleic acid or polypeptide sequences, includes reference to the residues in the two sequences which are the same when aligned. When percentage of sequence identity is used in reference to proteins, it is recognized that residue positions which are not identical often differ by conservative amino acid substitutions, where amino acid residues are substituted for other amino acid residues with similar chemical properties (*e.g.* charge or hydrophobicity) and therefore do not change the functional properties of the molecule. Where sequences differ in conservative substitutions, the percent sequence identity may be adjusted upwards to correct for the conservative nature of the substitution. Sequences which differ by such conservative substitutions are considered to have "sequence similarity" or "similarity". Means for making this adjustment are well-known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., according to the algorithm of Henikoff S and Henikoff JG (Amino acid substitution matrices from protein blocks. Proc. Natl. Acad. Sci. U.S.A. 1992, 89(22): 10915-9).

Identity can be determined using any homology comparison software, including for example, the BlastN software of the National Center of Biotechnology Information (NCBI) such as by using default parameters.

According to some embodiments of the invention, the identity is a global identity, i.e., an identity over the entire amino acid or nucleic acid sequences of the invention and not over portions thereof.

As used herein the term "query coverage" refers to a percentage that describes how much of the query sequence is covered by the target sequence.

As used herein, the terms "marker", "genomic marker" and "sub-genomic sequence" are used herein interchangeably and refer to a DNA (deoxyribonucleic acid) sequence present within the genome of a microbial strain.

According to certain exemplary embodiment, identity of a genomic marker sequence is defined as at least 90% query coverage with at least 95% identity, such as further described herein.

The terms "microbial strain(s)" and "bacterial strain(s)" are used herein interchangeably and refer to the bacterial strains of the invention as defined herein.

The terms "functional homolog", "functionally homologous", "variant" and grammatical equivalents are used herein interchangeably and refer to a modification (i.e., mutant, at least one mutation) of the bacterial strains of the invention resulting in a microbial strain that is endowed with substantially the same ensemble of biological activities, particularly anti-fungal and/or anti-oomycete activities (+/- 10%, 20%, 40%, 50%, or 60% when tested under the same conditions) as that of the strain of the invention and can be classified to the same species or strain based on known methods of species/strain classifications and as described herein. The modification can be man-made or evolutionary, e.g., during propagation with or without selection.

The term "isolated" refers to at least partially separated from the natural environment e.g., from the microbial strain habitat.

As used herein, the term "rhizoplane" refers to the external surface of roots together with closely adhering soil particles and debris.

As used herein, the term "rhizosphere" refers to the region of soil in the vicinity of plant roots, in which the soil chemistry and microbiology is influenced by the plant root growth, respiration, and nutrient exchange.

As used herein, the term "phyllosphere" refers to the total above-ground portions of a plant, which are a habitat for microorganisms. The phyllosphere can be further subdivided into the caulosphere (stems), phylloplane (leaves), anthosphere (flowers), and carposphere (fruit).

The present invention is based in part on a novel approach for the selection of microbial strains having fungicidal activity. Selection was guided by at least one of the following assumptions:
1) The plant microbiome is enriched with plant beneficial microorganisms, particularly bacteria, that co-evolved with plants and developed a mutualistic interaction with plants (Bulgarelli, D. et al. 2013. Structure and functions of the bacterial microbiota of plants. Annu. Rev. Plant Biol. 64:807-838).
2) Microbial strains that provide plants with functions that alleviate disease(s) are found in climatic zones, habitats and niches in which the plant is prone to experience attack by specific pathogens (Kindel LL., et al. A co-evolutionary framework for managing disease-suppressive soils. 2011 Annu. Rev. Phytopathol. 49:47-67).
3) Native plants co-evolved with the local microbial diversity exploit the available functional diversity for their survival and reproduction, and therefore are a better source for microbial strains with anti-phytopathogenic activity than non-native plants.

Selected strains were isolated and screened according to their ability to inhibit growth of phytopathogenic fungi/oomycetes causing plant diseases with negative effects on crop production. The isolated bacterial strains are described in Tables 1-3, and the strains' inhibiting activities are described in Tables 4-16 hereinbelow. Also contemplated are functional homologs of these strains, as defined and described herein.

According to certain embodiments, the bacterial strain or functional homolog thereof interacting with the host plant is present in the plant habitat, particularly in the rhizosphere (soil around root). According to certain embodiments, the bacterial strain or functional homolog thereof interacting with the host plant is present on or inside a plant tissue, including, but not limited to, the rhizoplane (root surface), root endosphere (inside the root), stem endosphere (inside the stem), leaf endosphere (inside the leaf), phyllosphere (on the shoot, stem and leaf surface), seed surface and seed endosphere (inside the seed). Each possibility represents a separate embodiment of the present invention.

According to one aspect, the present invention provides an isolated bacterial strain or a functional homologue thereof, wherein the isolated bacterial strain is selected from the group consisting of:
1) strain LAV49762, the strain being selected from the group consisting of
   a. a strain deposited under Accession Number 43430 at NCIMB;
   b. a strain comprising a 16S-rRNA sequence comprising the nucleic acid sequence set forth in SEQ ID NO:10;
   c. a strain comprising a genomic marker comprising the nucleic acid sequence set forth in any one of SEQ ID NOs:53-57;
   and any combination thereof;
2) strain LAV34085, the strain being selected from the group consisting of
   a. a strain deposited under Accession Number 43434 at NCIMB;
   b. a strain comprising a 16S-rRNA sequence comprising the nucleic acid sequence set forth in SEQ ID NO:1;
   c. a strain comprising a genomic marker comprising the nucleic acid sequence set forth in any one of SEQ ID NOs:21-25,
   and any combination thereof;
3) strain LAV43122, the strain being selected from the group consisting of
   a. a strain deposited under Accession Number 43431 at NCIMB;
   b. a strain comprising a 16S-rRNA sequence comprising a nucleic acid sequence selected from the group consisting of SEQ ID NO:2 and SEQ ID NO:3;
   c. a strain comprising a genomic marker comprising the nucleic acid sequence set forth in any one of SEQ ID NOs:26-27;
   and any combination thereof;
4) strain LAV43723, the strain being selected from the group consisting of
   a. a strain deposited under Accession Number 43436 at NCIMB;
   b. a strain comprising a 16S-rRNA sequence comprising the nucleic acid sequence set forth in SEQ ID NO:4;
   c. a strain comprising a genomic marker comprising the nucleic acid sequence set forth in any one of SEQ ID NOs:28-32;
   and any combination thereof;
5) strain LAV46348, the strain being selected from the group consisting of
   a. a strain deposited under Accession Number 43435 at NCIMB;
   b. a strain comprising a 16S-rRNA sequence comprising the nucleic acid sequence set forth in SEQ ID NO:5;
   c. a strain comprising a genomic marker comprising the nucleic acid sequence set forth in any one of SEQ ID NOs:33-37;
   and any combination thereof;
6) strain LAV49623, the strain being selected from the group consisting of
   a. a strain deposited under Accession Number 43429 at NCIMB;
   b. a strain comprising a 16S-rRNA sequence comprising the nucleic acid sequence set forth in SEQ ID NO:7;
   c. a strain comprising a genomic marker comprising the nucleic acid sequence set forth in any one of SEQ ID NOs:43-47;
   and any combination thereof;
7) strain LAV49648, the strain being selected from the group consisting of
   a. a strain deposited under Accession Number 43432 at NCIMB;
   b. a strain comprising a 16S-rRNA sequence comprising a nucleic acid sequence selected from the group consisting of SEQ ID NO:8 and SEQ ID NO:9;
   c. a strain comprising a genomic marker comprising the nucleic acid sequence set forth in any one of SEQ ID NOs:48-52;
   and any combination thereof;
8) strain LAV54823, the strain being selected from the group consisting of
   a. a strain deposited under Accession Number 43433 at NCIMB; and
   b. a strain comprising a 16S-rRNA sequence comprising the nucleic acid sequence set forth in SEQ ID NO:11;
   and a combination thereof;
9) strain LAV60069, the strain being selected from the group consisting of
   a. a strain deposited under Accession Number 43606 at NCIMB;
   b. a strain comprising a 16S-rRNA sequence comprising the nucleic acid sequence set forth in SEQ ID NO:16;
   c. a strain comprising a genomic marker comprising the nucleic acid sequence set forth in any one of SEQ ID NOs:67-71;
   and any combination thereof;
10) strain LAV61190, the strain being selected from the group consisting of
   a. a strain deposited under Accession Number 43607 at NCIMB;
   b. a strain comprising a 16S-rRNA sequence comprising the nucleic acid sequence set forth in SEQ ID NO:20;
   and a combination thereof;
11) strain LAV60070, the strain being selected from the group consisting of
   a. a strain deposited under Accession Number 43608 at NCIMB;
   b. a strain comprising a 16S-rRNA sequence selected from the group consisting of SEQ ID NO:17 and SEQ ID NO:18;
   and a combination thereof;
12) strain LAV60067, the strain being selected from the group consisting of
   a. a strain deposited under Accession Number 43609 at NCIMB;
   b. a strain comprising a 16S-rRNA sequence comprising the nucleic acid sequence set forth in SEQ ID NO:15;
   c. a strain comprising a genomic marker comprising the nucleic acid sequence set forth in any one of SEQ ID NOs:62-66;
   and any combination thereof;
13) strain LAV59924, the strain being selected from the group consisting of
   a. a strain deposited under Accession Number 43610 at NCIMB;
   b. a strain comprising a 16S-rRNA sequence comprising the nucleic acid sequence set forth in SEQ ID NO:12;
   and a combination thereof;
14) strain LAV48853, the strain being selected from the group consisting of
   a. a strain deposited under Accession Number 43611 at NCIMB;
   b. a strain comprising a 16S-rRNA sequence comprising the nucleic acid sequence set forth in SEQ ID NO:6;
   c. a strain comprising a genomic marker comprising the nucleic acid sequence set forth in any one of SEQ ID NOs:38-42;
   and any combination thereof;
15) strain LAV60063, the strain being selected from the group consisting of
   a. a strain deposited under Accession Number 43612 at NCIMB;
   b. a strain comprising a 16S-rRNA sequence comprising the nucleic acid sequence set forth in SEQ ID NO:13;
   c. a strain comprising a genomic marker comprising the nucleic acid sequence set forth in any one of SEQ ID NOs:58-59;
   and any combination thereof;
16) strain LAV60064, the strain being selected from the group consisting of
   a. a strain deposited under Accession Number 43613 at NCIMB;
   b. a strain comprising a 16S-rRNA sequence comprising the nucleic acid sequence set forth in SEQ ID NO:14;
   c. a strain comprising a genomic marker comprising the nucleic acid sequence set forth in any one of SEQ ID NOs:60-61;
   and any combination thereof; and
17) strain LAV60072, the strain being selected from the group consisting of
   a. a strain deposited under Accession Number 43614 at NCIMB;
   b. a strain comprising a 16S-rRNA sequence comprising the nucleic acid sequence set forth in SEQ ID NO:19;
   c. a strain comprising a genomic marker comprising the nucleic acid sequence set forth in SEQ ID NO:72;
   and any combination thereof.

Each possibility represents a separate embodiment of the present invention.

Bacterial strains of Accession numbers 43606, 43607, 43608, 43609, 43610, 43611, 43612, 43613, and 43614 were deposited at NCIMB Ltd., Ferguson Building, Craibstone Estate, Bucksburn, Aberdeen, AB21 9YA Scotland, on May 12, 2020. Bacterial strains of Accession numbers 43429, 43430, 43431, 43432, 43433, 43434 43435, and 43436 were deposited at NCIMB Ltd., Ferguson Building, Craibstone Estate, Bucksburn, Aberdeen, AB21 9YA Scotland, on July 5, 2019.

The criteria for identifying a functional homolog of a strain of the invention include functional and/or genetic criteria as are known to the skilled Artisan.

According to certain embodiments, the strain of the invention and the functional homolog belong to the same operational taxonomic units (OTU).

An "OTU"" (or plural, "OTUs") refers to a terminal leaf in a phylogenetic tree and is defined by a nucleic acid sequence, e.g., the entire genome or a specific genetic sequence, and all sequences that share sequence identity to this nucleic acid sequence at the level of species. According to certain exemplary embodiments, the specific genetic sequence may be the 16S-rRNA sequence or a portion of the 16S-rRNA (also referred to herein as "16S") sequence, or other functionally conserved sequences as listed below. According to additional exemplary embodiments, the entire genomes of two entities are sequenced and compared. In another embodiment, selected regions such as multilocus sequence tags (MLST, MLSA), specific genes, or sets of genes may be genetically compared. For16S-rRNA sequences, OTUs that share at least 97% average nucleotide identity across the entire 16S or some variable region of the 16S are considered the same OTU (see e.g. Claesson M J, et al. 2010. Nucleic Acids Res 38:e200; Konstantinidis K T, et al. 2006. Philos Trans R Soc Lond B Biol Sci 361:1929-1940). In embodiments involving the complete genome, MLSTs, specific genes, or sets of genes, OTUs that share at least 95% average nucleotide identity are considered the same OTU (see e.g. Achtman M, and Wagner M. 2008. Nat. Rev. Microbiol. 6:431-440; Konstantinidis et al. 2006, *ibid*)*.* OTUs are frequently defined by comparing sequences between organisms. Such characterization employs, e.g., whole genome sequencing (WGS) data.

According to certain embodiments, the functional homolog comprises a functionally conserved gene or a fragment thereof. According to certain embodiments, the functionally conserved gene is a house-keeping gene selected from the group consisting of, but not limited to, 16S-rRNA, *recA*, *glnII*, *atpD, gap, glnA*, *gltA*, *gyrB, pnp, rpoB*, *thrC* and *dnaK,* that is at least about 97%, at least about 98%, at least about 99%, at least about 99.1%, at least about 99.5%, at least about 99.6%, at least about 99.7%, at least about 99.8%, at least about 99.9% or more, or identical to that of a strain of the invention as described herein. Each possibility represents a separate embodiment of the present invention.

As mentioned, and according to a specific additional or an alternative embodiment, a functional homolog can also be determined on the basis of a multilocus sequence analysis (MLSA) determination of various functionally conserved genes or fragments thereof, e.g., at least one, 2, 3, 4, 5, 6, 7, 8, 9, 10, 11, 12, 13, 14, 15 or more functionally conserved genes or fragments thereof, such as of e.g., *16S*, *recA*, *glnII*, *atpD, gap, glnA*, *gltA*, *gyrB, pnp, rpoB, thrC and dnaK.*

According to certain exemplary embodiments, the house-keeping gene is 16S ribosomal RNA (16S-rRNA).

According to certain exemplary embodiments, the identity of the 16S sequence is defined as at least 100% query coverage with at least 97% identity.

According to these embodiments, the functional homolog bacterial strain comprises a 16S-rRNA sequence at least about 97%, at least about 97.1%, at least about 97.2%, at least about 97.3%, at least about 97.4%, at least about 97.5%, at least about 97.6%, at least about 97.7%, at least about 97.8%, at least about 97.9%, at least about 98%, at least about 98.1%, at least about 98.2%, at least about 98.3%, at least about 98.4%, at least about 98.5%, at least about 98.6%, at least about 98.7%, at least about 98.8%, at least about 98.9%, at least about 99%, at least about 99.1%, at least about 99.2%, at least about 99.%, at least about 99.4%, at least about 99.5%, at least about 99.6%, at least about 99.7%, at least about 99.8%, at least about 99.9% or more homologous, or identical to the 16S-rRNA sequence of a strain of the invention, said 16S-rRNA sequence of the strain of the invention comprises a nucleic acid sequence selected from the group consisting of SEQ ID NOs:1-20.

According to certain exemplary embodiments, the bacterial strain comprises more than one 16S-rRNA.

According to certain exemplary embodiments, the functional homolog of bacterial strain LAV49762 comprises a 16S-rRNA sequence at least 97% identical to SEQ ID NO:10.

According to certain exemplary embodiments, the functional homolog of bacterial strain LAV34085 comprises a 16S-rRNA sequence at least 97% identical to SEQ ID NO:1.

According to certain embodiments, the functional homolog of bacterial strain LAV43122 comprises a 16S-rRNA sequence at least 97% identical to any one of SEQ ID NO:2 and SEQ ID NO:3. According to some embodiments, the functional homolog of bacterial strain LAV43122 comprises a 16S-rRNA sequence at least 97.5%, at least 98%, at least 98.5% at least 99%, at least 99.5% or more identical to any one of SEQ ID NO:2 and SEQ ID NO:3. According to some embodiments, the functional homolog of bacterial strain LAV43122 comprises a 16S-rRNA sequence identical to any one of SEQ ID NO:2 and SEQ ID NO:3.

According to certain exemplary embodiments, the functional homolog of bacterial strain LAV43723 comprises a 16S-rRNA sequence at least 97% identical to SEQ ID NO:4.

According to certain exemplary embodiments, the functional homolog of bacterial strain LAV46348 comprises a 16S-rRNA sequence at least 97% identical to SEQ ID NO:5. According to some embodiments, the functional homolog of bacterial strain LAV43723 comprises a 16S-rRNA sequence at least 97.5%, at least 98%, at least 98.5% at least 99%, at least 99.5% or more identical to SEQ ID NO:5. According to some embodiments, the functional homolog of bacterial strain LAV46348 comprises a 16S-rRNA sequence having the nucleic acid sequence set forth in SEQ ID NO:5.

According to certain exemplary embodiments, the functional homolog of bacterial strain LAV49623 comprises a 16S-rRNA sequence at least 97% identical to SEQ ID NO:7.

According to certain exemplary embodiments, the functional homolog of bacterial strain LAV49648 comprises a 16S-rRNA sequence at least 97% identical to any one of SEQ ID NO:8 and SEQ ID NO:9. According to some embodiments, the functional homolog of bacterial strain LAV49648 comprises a 16S-rRNA sequence at least 97.5%, at least 98%, at least 98.5% at least 99%, at least 99.5% or more identical to any one of SEQ ID NO:8 and SEQ ID NO:9. According to some embodiments, the functional homolog of bacterial strain LAV49648 comprises a 16S-rRNA sequence having the nucleic acid sequence set forth in any one of SEQ ID NO:8 and SEQ ID NO:9.According to certain exemplary embodiments, the functional homolog of bacterial strain LAV54823 comprises a 16S-rRNA sequence at least 97% identical to SEQ ID NO:11. According to some embodiments, the functional homolog of bacterial strain LAV54823 comprises a 16S-rRNA sequence at least 97.5%, at least 98%, at least 98.5% at least 99%, at least 99.5% or more identical to SEQ ID NO:11. According to some embodiments, the functional homolog of bacterial strain LAV548238 comprises a 16S-rRNA sequence identical to SEQ ID NO:11.

According to certain exemplary embodiments, the functional homolog of bacterial strain LAV60069 comprises a 16S-rRNA sequence at least 97% identical to SEQ ID NO:16.

According to certain exemplary embodiments, the functional homolog of bacterial strain LAV61190 comprises a 16S-rRNA sequence at least 97% identical to SEQ ID NO:20.

According to certain exemplary embodiments, the functional homolog of bacterial strain LAV60067 comprises a 16S-rRNA sequence at least 97% identical to SEQ ID NO:15.

According to certain exemplary embodiments, the functional homolog of bacterial strain LAV59924 comprises a 16S-rRNA sequence at least 97% identical to SEQ ID NO:12.

According to certain exemplary embodiments, the functional homolog of bacterial strain LAV48853 comprises a 16S-rRNA sequence at least 97% identical to SEQ ID NO:6. According to some embodiments, the functional homolog of bacterial strain LAV48853 comprises a 16S-rRNA sequence at least 97.5%, at least 98%, at least 98.5% at least 99%, at least 99.5% or more identical to SEQ ID NO:6. According to some embodiments, the functional homolog of bacterial strain LAV48853 comprises a 16S-rRNA sequence identical to SEQ ID NO:6.

According to certain exemplary embodiments, the functional homolog of bacterial strain LAV60063 comprises a 16S-rRNA sequence at least 97% identical to SEQ ID NO: 13. According to some embodiments, the functional homolog of bacterial strain LAV60063 comprises a 16S-rRNA sequence at least 97.5%, at least 98%, at least 98.5% at least 99%, at least 99.5% or more identical to SEQ ID NO:13. According to some embodiments, the functional homolog of bacterial strain LAV60063 comprises a 16S-rRNA sequence identical to SEQ ID NO:13.

According to certain exemplary embodiments, the functional homolog of bacterial strain LAV60064 comprises a 16S-rRNA sequence at least 97% identical to SEQ ID NO:14.

According to certain exemplary embodiments, the functional homolog of bacterial strain LAV60072 comprises a 16S-rRNA sequence at least 97% identical to SEQ ID NO:19.

According to certain additional or alternative embodiments, the genomic nucleic acid sequences of the bacterial strain and the functional homolog thereof comprises at least one marker.

According to certain embodiments, the microbial strain of the present invention or the functional homolog thereof comprises at least two genomic markers, at least three genomic markers, at least four genomic markers, or at least five genomic markers.

According to certain exemplary embodiments, the functional homolog of a bacterial strain of the invention comprises at least two genomic markers selected from the group consisting of a marker having a nucleic acid sequence least about 95%, at least about 95.5%, at least about 96%, at least about 96.5%, at least about 97%, at least about 97.1%, at least about 97.2%, at least about 97.3%, at least about 97.4%, at least about 97.5%, at least about 97.6%, at least about 97.7%, at least about 97.8%, at least about 97.9%, at least about 98%, at least about 98.1%, at least about 98.2%, at least about 98.3%, at least about 98.4%, at least about 98.5%, at least about 98.6%, at least about 98.7%, at least about 98.8%, at least about 98.9%, at least about 99%, at least about 99.1%, at least about 99.2%, at least about 99.3%, at least about 99.4%, at least about 99.5%, at least about 99.6%, at least about 99.7%, at least about 99.8%, at least about 99.9% or more homologous, or identical to any one of SEQ ID NOs:21-72.

According to certain embodiments, the functional homolog of bacterial strain LAV49762 comprises a marker having at least 95% local identity to a nucleic acid sequence set forth in any one of SEQ ID NOs:53-57 over 90% coverage.

According to certain exemplary embodiments, the functional homolog of bacterial strain LAV34085 comprises a marker having at least 95% local identity to a nucleic acid sequence set forth in any one of SEQ ID NOs:21-25 over 90% coverage.

According to certain exemplary embodiments, the functional homolog of bacterial strain LAV43122 comprises a marker having at least 95% local identity to a nucleic acid sequence set forth in any one of SEQ ID NOs:26-27 over 90% coverage.

According to certain exemplary embodiments, the functional homolog of bacterial strain LAV43723 comprises a marker having at least 95% local identity to a nucleic acid sequence set forth in any one of SEQ ID NOs:28-32 over 90% coverage.

According to certain exemplary embodiments, the functional homolog of bacterial strain LAV46348 comprises a marker having at least 95% local identity to a nucleic acid sequence set forth in any one of SEQ ID NOs:33-37 over 90% coverage.

According to certain embodiments, the functional homolog of bacterial strain LAV48853 comprises a marker having at least 95% local identity to a nucleic acid sequence set forth in any one of SEQ ID NOs:38-42 over 90% coverage.

According to certain exemplary embodiments, the functional homolog of bacterial strain LAV49623 comprises a marker having at least 95% local identity to a nucleic acid sequence set forth in any one of SEQ ID NOs:43-47 over 90% coverage

According to certain exemplary embodiments, the functional homolog of bacterial strain LAV49648 comprises a marker having at least 95% local identity to a nucleic acid sequence set forth in any one of SEQ ID NOs:48-52 over 90% coverage.

According to certain exemplary embodiments, the functional homolog of bacterial strain LAV60063 comprises a marker having at least 95% local identity to a nucleic acid sequence set forth in any one of SEQ ID NOs:58-59 over 90% coverage.

According to certain exemplary embodiments, the functional homolog of bacterial strain LAV60064 comprises a marker having at least 95% local identity to a nucleic acid sequence set forth in any one of SEQ ID NOs:60-61 over 90% coverage.

According to certain exemplary embodiments, the functional homolog of bacterial strain LAV60067 comprises a marker having at least 95% local identity to a nucleic acid sequence set forth in any one of SEQ ID NOs:62-66 over 90% coverage.

According to certain exemplary embodiments, the functional homolog of bacterial strain LAV60069 comprises a marker having at least 95% local identity to a nucleic acid sequence set forth in any one of SEQ ID NOs:67-71 over 90% coverage.

According to certain exemplary embodiments, the functional homolog of bacterial strain LAV60072 comprises a marker having at least 95% local identity to the nucleic acid sequence set forth in SEQ ID NO:72 over 90% coverage.

According to certain embodiments, the classification is based on DNA-DNA pairing data and/or sequence identity to functionally conserved genes or fragments thereof.

According to certain embodiments, a species/strain can be defined by DNA-DNA hybridization involving a pairwise comparison of two entire genomes, and reflecting the overall sequence similarity between them. According to certain exemplary embodiments, a species is defined as a set of strains with at least about 70%, e.g., at least about 75%, at least about 80%, at least about 85%, at least about 90%, at least about 91%, at least about 92%, at least about 93%, at least about 94%, at least about 95%, at least about 96%, at least 97% or more DNA-DNA relatedness and with 5°C or less ΔTm (Wayne et al. 1987, Int J Syst Evol Microbiol 37:463) and having anti-fungal and/or anti-oomycete activity.

According to certain exemplary embodiments, a strain of the invention and a functional homolog thereto have at least about 97%, at least about 97.1%, at least about 97.2%, at least about 97.3%, at least about 97.4%, at least about 97.5%, at least about 97.6%, at least about 97.7%, at least about 97.8%, at least about 979 %, at least about 98%, at least about 98.1%, at least about 98.2%, at least about 98.3%, at least about 98.4%, at least about 98.5%, at least about 98.6%, at least about 98.7%, at least about 98.8%, at least about 98.9%, at least about 99%, at least about 99.1%, at least about 99.2%, at least about 99.3%, at least about 99.4%, at least about 99.5%, at least about 99.6%, at least about 99.7%, at least about 99.8%, at least about 99.9%, or 100% DNA-DNA relatedness and with 5°C or less ΔTm and having anti-fungal and/or anti-oomycete activities as described herein.

According to certain embodiments, the degree of DNA-DNA relatedness between the functional homolog and the strain of the invention is determined as the Tetranucleotide Signature Frequency Correlation Coefficient, which is based on oligonucleotide frequencies (Bohlin J. et al. 2008, BMC Genomics, 9:104). In some embodiments, the Tetranucleotide Signature Frequency Correlation coefficient between the variant and the strain of the invention is of about 0.99, 0.999, 0.9999, 0.99999, 0.999999, 0.9999999 or more.

According to an additional or alternative embodiment, the degree of relatedness between the functional homolog and the strain of the invention is determined as the degree of similarity obtained when analyzing the genomes of the parent and of the variant strain by Pulsed-field gel electrophoresis (PFGE) using one or more restriction endonucleases. The degree of similarity obtained by PFGE can be measured by the Dice similarity coefficient. In some embodiments, the Dice similarity coefficient between the variant and the deposited strain is of at least about 96%, at least about 97%, at least about 98%, at least about 99%, at least about 99%, at least about 99.5%, at least about 99.6%, at least about 99.7%, at least about 99.8%, at least about 99.9%, or more.

According to certain embodiments, the genomic nucleic acid sequence of a functional homolog is at least about 97%, at least about 97.1%, at least about 97.2%, at least about 97.3%, at least about 97.4%, at least about 97.5%, at least about 97.6%, at least about 97.7%, at least about 97.8%, at least about 979 %, at least about 98%, at least about 98.1%, at least about 98.2%, at least about 98.3%, at least about 98.4%, at least about 98.5%, at least about 98.6%, at least about 98.7%, at least about 98.8%, at least about 98.9%, at least about 99%, at least about 99.1%, at least about 99.2%, at least about 99.3%, at least about 99.4%, at least about 99.5%, at least about 99.6%, at least about 99.7%, at least about 99.8%, at least about 99.9% or more, or identical to the genomic nucleic acid sequence of a strain of the present invention.

According to additional or alternative embodiments, the functional homolog is defined as having the same ribotype, as obtained using any of the methods known in the art and described, for instance, by Bouchet et al. (Clin. Microbiol. Rev., 2008, 21:262-273).

According to additional or alternative embodiments, the deposited strain and the functional homolog are characterized by substantially the same (+/- about 10%, 20%, 40%, 50%, 60% when tested under the same conditions) biochemical profiling (e.g., biochemical fingerprinting) using for example, the GEN III redox chemistry (BIOLOG Inc. 21124 Cabot Blvd. Hayward CA, USA), which can analyze both Gram-negative and Gram-positive bacteria, for their ability to metabolize all major classes of biochemicals, in addition to determining other important physiological properties such as pH, salt, and lactic acid tolerance. Further details of biochemical profiling can be obtained in "Modern Phenotypic Microbial Identification", Bochner B.R., Encyclopedia of Rapid Microbiological Methods, 2006, v.2, Ch. 3, pp. 55-73.

According to additional or alternative embodiments, the functional homolog is defined by a comparison of coding sequence (gene) order.

According to further additional or alternative embodiments, the functional homolog is defined by a comparison of non-coding sequence order.

According to yet additional or alternative embodiments, the functional homolog is defined by a comparison of coding and non-coding sequence order.

As used herein, "combined coding region" refers to a nucleic acid sequence including all of the coding regions of the bacterial isolate, yet without the non-coding regions of the bacterial isolate.

According to some embodiments of the invention, the combined coding region of the functional homolog is such that it maintains the original order of the coding regions as within the genomic sequence of the bacterial isolate. The non-coding regions may be the same, different or absent.

For example, in case the genomic sequence has the following coding regions: A, B, C, D, E, F, G, each flanked by non-coding sequences (e.g., regulatory elements, introns and the like), the combined coding region will include a single nucleic acid sequence having the A+B+C+D+E+F+G coding regions combined together, while maintaining the original order of their genome, yet without the non-coding sequences. The coding regions may be the same, different or absent.

According to some embodiments of the invention, the combined non-coding region of the functional homolog is such that it maintains the original order of the non-coding regions as within the genomic sequence of the bacterial isolate.

According to some embodiments of the invention, the combined non-coding region and coding region (i.e., the genome) of the functional homolog is such that it maintains the original order of the coding and non-coding regions as within the genomic sequence of the microbial deposit.

As used herein, the term "maintain" refers to keeping at least about 90%, 9 %, 92%, 93%, 94%, 95%, 96%, 97%, 98%, 99% or 100% of the characteristic (e.g. coding sequence order) as compared to the deposited strain.

According to additional or alternative embodiments, the functional homolog is defined by a comparison of gene content.

According to a specific embodiment, the functional homolog comprises a combined coding region comprising a nucleic acid sequence at least about 90%, at least about 91%, at least about 92%, at least about 93%, at least about 94%, at least about 95%, at least about 96%, at least about 97%, at least about 98%, at least about 99%, or more (e.g., 100%) identical to a combined coding region existing in the genome of the deposited strain.

According to additional or alternative embodiments, the functional homolog is defined by a comparison of nucleotide composition and codon usage.

According to additional or alternative embodiments, the functional homolog is defined by a method based on random genome fragments and DNA microarray technology. These methods are of sufficiently high resolution for strain-to-species level identification.

One of ordinary skill in the art, based on knowledge of the classification criteria and on embodiments described herein, would know how to identify strains that are considered functional homologs.

An additional and more detailed description of species-to-strain classification can be found in: Cho and Tiedje, 2001. Appl. Environ Microbiol. 67:3667-82; Coenye et al., 2005. FEMS Microbiol. Rev. 29:147-167; Konstantinidis and Tiedje, 2005. Proc. Natl. Acad. Sci. USA 102:189-197; Konstantinidis et al., 2006. Appl. Environ. Microbiol. 72:7286-7293).

It is to be understood that one or more methods as described herein can be used to identify a functional homolog.

Genomic data can be obtained by methods which are well known in the art, e.g., DNA sequencing, bioinformatics, electrophoresis, enzyme-based mismatch detection assay and a hybridization assay; including, e.g. PCR, RT-PCR, RNase protection, in-situ hybridization, primer extension, Southern blot, Northern Blot and dot blot analysis and the like.

According to certain exemplary embodiments, the functional homolog and the strain belong to the same genus.

According to certain exemplary embodiments, the strains of the invention are of a genus selected from the group consisting of *Pseudomonas, Serratia, Streptomyces, Pantoea, Enterobacter, Gluconobacter, Bacillus* and *Erwinia.* Each possibility represents a separate embodiment of the present invention.

According to certain embodiments, strain LAV34085 is of the genus *Serratia,* particularly of the species *Serratia plymutica.*

According to certain embodiments, strain LAV49762, strain LAV43122, LAV46348, strain LAV49762, strain LAV48853, strain LAV60063, strain LAV60064, and strain LAV60072 are each of the genus *Pseudomonas.* According to certain exemplary embodiments, strain LAV46348, strain LAV48853 and strain LAV60063 are of the species *Pseudomonas fluorescens.* According to certain exemplary embodiments, strain LAV43122 is of the species *Pseudomonas chlororaphis.* According to certain exemplary embodiments, strain LAV49762 is of the species *Pseudomonas rhizosphaerae.* According to certain exemplary embodiments, strain LAV60064 is of the species *Pseudomonas psychrotolerans.* According to further certain exemplary embodiments, strain LAV60072 is of the species *Pseudomonas azotoformans.*

According to certain embodiments, strain LAV43723 is of the genus *Streptomyces,* particularly of the species *Streptomyces fulvissimus.*

According to certain embodiments, strain LAV49623 and strain LAV59924 are each of the genus *Pantoea.* According to certain exemplary embodiments, strain LAV49623 is of the species *Pantoea vagans.* According to certain exemplary embodiments, strain LAV59924 is of the species *Pantoea agglomerans.*

According to certain embodiments, strain LAV49648 and strain LAV60070 are each of the genus *Enterobacter,* particularly of the species *Enterobacter cloacae.*

According to certain embodiments, strain LAV54823 is of the genus *Erwinia,* particularly of the species *Erwinia gerundensis.*

According to certain embodiments, strain LAV60069 and strain LAV61190 are each of the genus *Bacillus.* According to certain exemplary embodiments, strain LAV60069 is of the species *Bacillus endophyticus.* According to certain exemplary embodiments, strain LAV61190 is of the species *Bacillus velezensis.*

According to certain embodiments, strain LAV60067 is of the genus *Gluconobacter,* particularly of the species *Gluconobacter frateurii.*

According to certain exemplary embodiments, the functional homolog and the strain of the invention belong to the same species.

According to certain exemplary embodiments, the functional homolog and the strain of the invention belong to the same sub-species.

As used herein, the term "preparation" refers to an isolate of bacteria in which the prevalence (i.e., concentration and/or ratio) of the bacterial strain or functional homolog is enriched over that (exceeds that) found in nature. In nature, the bacterial strain is typically part of the plant microbiome, consisting of more than thousands of microbial species, whether in the phyllosphere, including endophytes, rhizoplane, rhizosphere or any other plant compartment. According to some embodiments of the invention, the preparation comprises less than 50, 20, 10, 9, 8, 7, 6, 5, or 4 microbial species, e.g., bacteria and fungi.

According to certain exemplary embodiments, the microbial preparations comprise 1, 2, 3, 4, 5, 6, 7, 8, 9 or 10 microbial species. Each possibility represents a separate embodiment of the present invention.

According to further exemplary embodiments, the microbial preparation comprises a single microbial species, i.e. bacteria according to the teachings of the present invention.

According to certain exemplary embodiments, the preparation comprises a single strain of the present invention, wherein said preparation is devoid of other microbial species. According to certain further exemplary embodiments, the preparation comprises a plurality of strains of the present invention, wherein said preparation is devoid of other microbial species.

According to certain embodiments, the preparation comprises the bacterial strain of the invention at a level of purity of at least about 20%, at least about 30%, at least about 40%, at least about 50%, at least about 60%, at least about 70%, at least about 80%, at least about 85%, at least about 90%, at least about 91%, at least about 92%, at least about 93%, at least about 94%, at least about 95% or more, say 100% pure. As used herein, the term "pure" or "purity" refers to the percentage of the bacterial strain of the invention out of the total number of microorganisms in the preparation.

According to certain exemplary embodiments, the preparation comprises the bacterial strain of the invention at a level of purity of at least about 99%, at least about 99.1%, at least about 99.2%, at least about 99.3%, at least about 99.4%, at least about 99.5%, at least about 99.6%, at least about 99.7%, at least about 99.8%, at least about 99.9%, at least about 99.95%, at least about 99.99%, at least about 99.99%, at least about 99.999% or more, say 100% pure.

According to certain embodiments, the bacterial preparation comprises viable bacterial cells (capable of replicating). According to some embodiments, the bacterial strain comprises sporulating bacteria.

According to some embodiments, the bacterial preparations of the invention comprises non-viable forms of the bacterial strains of the invention. According to certain embodiments, the non-viable form comprises non-sporulating bacteria. According to some embodiments, the non-viable form comprises dried bacterial strains.

A "spore" or "spores" refers to microbial structures that are generally viable, more resistant to environmental influences such as heat and bactericidal agents than other forms of the same microbial species, and typically capable of germination and out-growth. Bacteria that are "capable of forming spores" are those bacteria comprising the genes and other necessary abilities to produce spores under suitable environmental conditions. As used herein, the term "enriched" refers to 2-10×10⁶-fold enrichment over that found in nature in an isolate of microbiota obtained from a plant phyllosphere and/or rhizosphere comprising a strain of the invention or a functional homolog of same.

As used herein, the phrase "CFUs" or "Colony Forming Units" refers to the number of microbial cells in a defined sample (e.g. milliliter of liquid, square centimeter of surface, one seed of grain, etc.) that form colonies and thereafter numbered, on a semi-solid bacteriological growth medium.

According to certain embodiments, the concentration in the composition e.g., preparation, formulation, coated seed etc. is 10² CFUs-10⁹ CFUs/ seed or 10² CFUs-10⁹ CFUs/ gr powder or 10² CFUs-10⁹ CFUs/ ml.

According to certain embodiments, the preparation is in a form selected from the group consisting of a liquid culture, a still (plate or non-shaking liquid) culture, whole culture stored stock of cells (particularly glycerol stocks), agar strip, stored agar plug in glycerol/water, freeze dried stock, and dried stocks such as lyophilizate dried onto filter paper or grain seed.

As used herein, the term "culture" refers to a fluid, pellet, scraping, dried sample, lyophilizate or a support, container, or medium such as a plate, paper, filter, matrix, straw, pipette or pipette tip, fiber, needle, gel, swab, tube, vial, particle, etc. that contains the deposited strain or the functional homolog thereof in an amount that exceeds that found in nature, as described hereinabove. In the present invention, an "isolated culture" of a microbial strain is a culture fluid or a scraping, pellet, dried preparation, lyophilizate, or a support, container, or medium that contains the deposited strain or the functional homolog thereof, in the absence of other microorganisms.

Cultures of the deposited strains or of functional homologs thereof may be prepared for use according to the teachings of the invention using standard fermentation techniques known in the art. Growth is commonly performed in a bioreactor.

A bioreactor refers to any device or system that supports a biologically active environment. As described herein, a bioreactor is a vessel in which microorganisms, including the microorganism of the invention, can be grown. A bioreactor may be any appropriate shape or size for growing the microorganisms. A bioreactor may range in size and scale from 10 mL (e.g., small scale) to liters to cubic meters (e.g., large scale) and may be made of stainless steel, disposable material (e.g., nylon, plastic bags) or any other appropriate material as known and used in the art. The bioreactor may be a batch type bioreactor, a fed batch type or a continuous-type bioreactor (e.g., a continuous stirred reactor). For example, a bioreactor may be a chemostat as known and used in the art of microbiology for growing and harvesting microorganisms. A bioreactor may be obtained from any commercial supplier (See also Bioreactor System Design, Asenjo and Merchuk, CRC Press, 1995).

For small scale operations, a batch bioreactor may be used, for example, to test and develop new processes, and for processes that cannot be converted to continuous operations.

Microorganisms grown in a bioreactor may be suspended or immobilized. Growth in the bioreactor is generally under aerobic conditions at suitable temperatures and pH for growth. For the organisms of the invention, cell growth can be achieved at temperatures between 5-37°C., with an exemplary temperature range selected from 15 to 30°C, 15 to 28°C, 20 to 30°C, or 15 to 25°C. The pH of the nutrient medium can vary between 4.0 and 9.0. For example, the operating range can be usually slightly acidic to neutral at pH 4.0 to 7.0, or 4.5 to 6.5, or pH 5.0 to 6.0. Typically, maximal cell yield is obtained in 20-72 hours after inoculation.

Optimal conditions for the cultivation of the microorganisms of this invention will, of course, depend upon the particular strain and strain species. However, by virtue of the conditions applied in the selection process and general requirements of most microorganisms, a person of ordinary skill in the art would be able to determine essential nutrients and conditions. The microorganisms would typically be grown in aerobic liquid cultures on media which contain sources of carbon, nitrogen, and inorganic salts that can be assimilated by the microorganism and supportive of efficient cell growth. Exemplary carbon sources are hexoses such as glucose, but other sources that are readily assimilated, such as amino acids, may form a substitute. Many inorganic and proteinaceous materials may be used as nitrogen sources in the growth process. Exemplary nitrogen sources are amino acids and urea, but others include gaseous ammonia, inorganic salts of nitrate and ammonium, vitamins, purines, pyrimidines, yeast extract, beef extract, proteose peptone, soybean meal, hydrolysates of casein, distiller's solubles, and the like. Among the inorganic minerals that can be incorporated into the nutrient medium are the customary salts capable of yielding calcium, zinc, iron, manganese, magnesium, copper, cobalt, potassium, sodium, molybdate, phosphate, sulfate, chloride, borate, and like ions.

The culture can be a pure culture, whereby a single microbial strain is grown, or a mixed culture. A mixed culture can be prepared pending the compliance of the microbial strains to co-exist and proliferate under the same culturing conditions. When needed, an antibiotic or other growth-restricting conditions, e.g., temperature, essential nutrients and the like can be employed during culturing to restrict the growth of other microorganisms (contaminants) not desired in the culture/co-culture.

According to alternative or additional embodiments, a desired strain combination is produced following culturing. Typically, a strain combination is made after culturing when the microbial strains do not share the same or optimal culturing conditions.

When a combination of strains is to be used, the ratio of each type of microorganism in the final product will depend on the target fungi/oomycetes to be eradicated.

It is to be explicitly understood that the present invention encompasses complete cultures comprising a growth medium and at least one bacterial strains or functional homologs of the invention, as well as growth medium obtained after removal of the bacterial strains. According to certain embodiments, the growth medium obtained after removal of the at least one bacterial strain encompasses fermentation products of said at least one bacterial cell.

According to a further aspect, the present invention provides a lysate of at least one bacterial strain or of a functional homolog thereof according to the teachings of the present invention.

According to the certain embodiments, the lysate is of a single strain. According to some embodiments, the lysate is of a plurality of strains. According to certain embodiments, the plurality of strains is of the same genus and/or species. According to some embodiments, the plurality of strains comprises a plurality of strain genera and/or species.

According to certain embodiments, the lysate comprises a whole cell lysate of the bacteria.

According to certain embodiments, the lysate comprises a soluble fraction of the bacterial cells.

According to a further aspect, the present invention provides a cell extract of at least one bacterial strain or a functional homolog thereof according to the teachings of the present invention. According to some embodiments, the cell extract is of a plurality of strains. According to certain embodiments, the plurality of strains is of the same genus and/or species. According to some embodiments, the plurality of strains comprises a plurality of strain genera and/or species.

According to an additional aspect, the present invention provides a composition comprising the bacterial preparation, culture, culture medium, lysate or extract as described herein, further comprising agriculturally acceptable carriers and/or diluents.

According to certain embodiments, the composition further comprises an agriculturally effective amount of an active agent selected from the group consisting of a fertilizer, an acaricide, a bactericide, a fungicide, an insecticide, a microbicide, a nematicide, a pesticide, a plant growth regulator, a rodenticide, a nutrient.

Also provided is a formulation comprising the bacterial preparation or the composition comprising same as described herein.

Any carrier suitable for agricultural use can form part of the compositions and/or formulations of the present invention. The carrier may be any one or more of a number of carriers that confer a variety of properties, including increased stability, wettability, dispersability, etc. Wetting agents such as natural or synthetic surfactants, which can be nonionic or ionic surfactants, or a combination thereof can be included in a composition of the invention. Water-in-oil emulsions can also be used to formulate a composition that includes at least one isolated microorganism of the present invention (see, for example, U.S. Patent No. 7,485,451). Suitable formulations that may be prepared include wettable powders, granules, gels, agar strips or pellets, and the like, microencapsulated particles, and the like, liquids such as aqueous flowables, aqueous suspensions, water-in-oil emulsions, etc. The formulation may include grain or legume products (e.g., ground grain or beans, broth or flour derived from grain or beans), starch, sugar, or oil. The carrier may be an agricultural carrier. In certain preferred embodiments, the carrier is a seed, and the composition may be applied or coated onto the seed or allowed to saturate the seed.

According to some embodiments, the agricultural carrier may be soil or plant growth medium. Other agricultural carriers that may be used include water, plant-based oils, humectants, or combinations thereof. Alternatively, the agricultural carrier may be a solid, such as diatomaceous earth, loam, silica, alginate, clay, bentonite, vermiculite, seed cases, other plant and animal products, or combinations, including granules, pellets, or suspensions. Mixtures of any of the aforementioned ingredients are also contemplated as carriers, such as but not limited to, pesta (flour and kaolin clay), agar or flour-based pellets in loam, sand, clay, etc. Formulations may include food sources for the cultured organisms, such as barley, rice, or other biological materials such as seed, plant parts, sugar cane bagasse, hulls or stalks from grain processing, ground plant material ("yard waste") or wood from building site refuse, sawdust or small fibers from recycling of paper, fabric, or wood. Other suitable formulations will be known to those skilled in the art.

In the liquid form, e.g., solutions or suspensions, the microbial strain may be mixed or suspended in water or in aqueous solutions. Suitable liquid diluents or carriers include water, aqueous solutions, petroleum distillates, or other liquid carriers.

Solid compositions can be prepared by dispersing the microbial strain in and on an appropriately divided solid carrier, such as peat, wheat, bran, vermiculite, clay, talc, bentonite, diatomaceous earth, fuller's earth, pasteurized soil, and the like. When such formulations are used as wettable powders, biologically compatible dispersing agents such as non-ionic, anionic, amphoteric, or cationic dispersing and emulsifying agents can be used.

According to certain embodiments, the bacterial strains of the present invention are applied within a "dehydrated microfermentor" as described in International (PCT) Application No. PCT/IL2019/050838. As used herein, this term refers to a dehydrated composition comprising a particle encapsulating one or more microorganisms, wherein the particle is composed of an inner core comprising the one or more microorganisms surrounded by an outer shell layer, wherein said outer shell layer is selectively permeable to a rehydrating fluid, and wherein upon fluid absorption, said outer shell layer degrades at a predetermined rate, thereby releasing a plurality of microorganisms to the surrounding environment in a controlled manner. According to certain embodiments, the encapsulated microorganisms, particularly the bacterial strains and functional homologs of the invention, are present in the dehydrated composition at an initial concentration of less than about 1×10³ CFU, and, following fluid absorption, the concentration of the encapsulated microorganisms is increased by at least 10-fold before the microorganisms are released to the surrounding environment. According to certain exemplary embodiments, the concentration of the released microorganisms is at least 10⁵ CFU. In certain embodiments, the dehydrated composition comprises a plurality of particles.

Any fertilizer as is known in the art can be added to the compositions/formulations of the present invention, as long as the fertilizer does not interfere with the bacterial growth and activity. According to certain embodiments, the fertilizer is selected from the group consisting of chemical or biological fertilizer. The amount of the at least one chemical or biological fertilizer employed can vary depending on the final formulation as well as the size of the plant and/or seed to be treated.

A variety of chemical pesticides is apparent to one of skill in the art and may be used. Exemplary chemical pesticides include acylalanines, butyrolactones, oxazolidinones, hydroxy-(2-amino-) pyrimidines, isothiazolones, isoxazoles, carboxylic acids, benzimidazoles, thiophanates, N-phenyl carbamates, toluamides, ethylamino-thiazole-carboxamide, phenylureas, pyridinylmethyl-benzamides, aminocyanoacrylates, benzophenone, benzoylpyridine, pyrazole-5-carboxamides, pyrimidinamines, quinazoline, N-methoxy-(phenyl-ethyl)-pyrazole-carboxamides, furan-carboxamides, oxathiin-carboxamides, phenyl-benzamides, phenyl-oxo-ethyl thiophene amide, pyrazole-4-carboxamides, N-cyclopropyl-N-benzyl-pyrazole-carboxamides, pyridine-carboxamides, pyridinyl-ethyl-benzamides, thiazole-carboxamides, pyrazine-carboxamides, benzyl-carbamates, dihydro-dioxazines, imidazolinones, methoxyacetamide, methoxy-acrylates, methoxy-carbamates, oxazolidine-diones, oximino-acetamides, oximino-acetates, tetrazolinones, cyano-imidazole, sulfamoyl-triazole, picolinamides, dinitrophenyl crotonates, 2,6-dinitro-anilines, tri-phenyl tin compounds, thiophene-carboxamides, triazolo-pyrimidylamine, anilino-pyrimidines, enopyranuronic acid, hexopyranosyls, glucopyranosyls, tetracycline, aryloxyquinoline, quinazolinone, phenylpyrroles, dicarboximides, dithiolanes, phosphoro-thiolates, aromatic hydrocarbons, 1,2,4-thiadiazoles, carbamates, piperidinyl-thiazole-isoxazolines, imidazoles, piperazines, pyridines, pyrimidines, triazoles, triazolinthiones, morpholines, piperidines, spiroketal-amines, amino-pyrazolinone, hydroxyanilides, allylamines, thiocarbamates, peptidyl pyrimidine nucleoside, cinnamic acid amides, mandelic acid amides, valinamide carbamates, isobenzo-furanone, pyrrolo-quinolinone, triazolobenzo-thiazole, carboxamide, cyclopropane-carboxamide, propionamide, trifluoroethyl-carbamate, benzothiadiazole, thiadiazole-carboxamide, ethyl phosphonates, cyanoacetamide-oxime, phthalamic acids, benzotriazines, benzene-sulfonamides, pyridazinones, phenyl-acetamide, guanidines, cyano-methylene-thiazolidines, pyrimidinone-hydrazones, 4-quinolyl-acetates, tetrazolyloximes, dithiocarbamates, phthalimides, chloronitriles, sulfamides, bis-guanidines, triazines, quinones, quinoxalines, maleimide and phthalonitriles, as well as copper fungicides (acypetacs-copper, Bordeaux mixture, Burgundy mixture, Cheshunt mixture, copper acetate, basic copper carbonate, copper hydroxide, copper naphthenate, copper oleate, copper oxychloride, copper silicate, copper sulfate, basic copper sulfate, copper zinc chromate, cufraneb, cuprobam, cuprous oxide, mancopper, oxine-copper, saisentong, thiodiazole-copper) and other fungicides, including dimethomorph, fosetyl-Al, tetraconazole, azoxystrobin, propineb, pyraclostrobin, potassium phosphite, mefenoxam, and folpet.

The formulation as used herein can also refer to a customary formulation in an effective amount to be applied either to the soil (i.e., in-furrow), to a portion of the plant (i.e., drench) or on the seed before planting (i.e., seed coating or dressing). Customary formulations include solutions, emulsifiable concentrates, wettable powders, suspension concentrates, soluble powders, granules, suspension-emulsion concentrates, natural and synthetic materials impregnated with active compounds, and very fine controlled release capsules in polymeric substances. In certain embodiments of the present invention, the microbial strains are formulated in powders that are available in either a ready-to-use formulation or are otherwise mixed together at the time of use. In either embodiment, the powder may be admixed with the soil prior to or at the time of planting.

Depending on the final formulation, one or more suitable additives can also be introduced to the compositions of the present invention. Adhesives such as carboxymethylcellulose and natural and synthetic polymers in the form of powders, granules or latexes, such as gum arabic, chitin, polyvinyl alcohol and polyvinyl acetate, as well as natural phospholipids, such as cephalins and lecithins, and synthetic phospholipids, can be added to the compositions/formulation of the present invention.

According to certain embodiments, the bacterial strains are formulated in a single, stable solution, or emulsion, or suspension. For solutions, the chemical compounds are typically dissolved in solvents before the microbial strain is added. Suitable liquid solvents include petroleum based aromatics, such as xylene, toluene or alkylnaphthalenes, aliphatic hydrocarbons, such as cyclohexane or paraffins, for example petroleum fractions, mineral and vegetable oils, alcohols, such as butanol or glycol as well as their ethers and esters, ketones, such as methyl ethyl ketone, methyl isobutyl ketone or cyclohexanone, strongly polar solvents, such as dimethylformamide and dimethyl sulphoxide. For emulsion or suspension, the liquid medium is water. In some embodiments, the chemical agent and the microbial strain are suspended in separate liquids and mixed at the time of application. In a preferred embodiment of suspension, the chemical agent and the microbial strain are combined in a ready-to-use formulation that exhibits a reasonably long shelf-life. In use, the liquid can be sprayed or can be applied to the plant foliar as an atomized spray or in-furrow at the time of planting the crop. The liquid composition can be introduced in an effective amount on the seed (i.e., seed coating or dressing) or to the soil (i.e., in-furrow) before germination of the seed or directly to the soil in contact with the roots by utilizing a variety of techniques known in the art including, but not limited to, drip irrigation, sprinklers, soil injection or soil drenching. Optionally, stabilizers and buffers can be added, including alkaline and alkaline earth metal salts and organic acids, such as citric acid and ascorbic acid, inorganic acids, such as hydrochloric acid or sulfuric acid. Biocides can also be added and can include formaldehydes or formaldehyde-releasing agents and derivatives of benzoic acid, such as p-hydroxybenzoic acid.

The amount of the bacterial strain or functional homolog within the composition/formulation is sufficient to interact, colonize and/or localize in a cultivated plant treated with same.

One of ordinary skill in the art would know how to calculate the concentration of the microbial strain or functional homolog.

According to certain embodiments, the bacterial strain(s) is about 2% w/w to about 80% w/w of the entire formulation/composition. According to other embodiments, the bacterial strains(s) employed in the compositions is about 5% w/w to about 65% w/w or about 10% w/w to about 60% w/w by weight of the entire formulation/composition.

According to certain embodiments, the preparation/composition provided herein is formulated to provide stability for the bacterial strain or functional homolog. Optionally, a shelf-stable formulation is in a dry form, e.g. a powder formulation, or a lyophilized formulation. According to certain embodiments, the formulation is substantially stable at temperatures between about -20°C and about 50°C for at least about 1, 2, 3, 4, 5, or 6 days, or 1, 2, 3 or 4 weeks, or 1, 2, 3, 4, 5, 6, 7, 8, 9, 10, 11 or 12 months, or one or more years. In other embodiments, the formulation is substantially stable at temperatures between about 4°C and about 37°C for at least about 5, 10, 15, 20, 25, 30 or greater than 180 days. According to certain exemplary embodiments, the microbial strain or functional homolog may be shelf-stable, wherein at least 0.01% of the CFU or spores are viable after storage in desiccated form (i.e., moisture content of 30% or less) for 1, 2, 3, 4, 5, 6, 7, 8, 9, 10 or more than 10 weeks at 4°C or at room temperature.

The bacterial strains of the present invention and their functional homologs are effective in conferring resistance to plants contacted with the strain preparations or compositions/formulations comprising same.

As used herein, the terms "conferred resistance to a pathogenic fungus and/or oomycete" or "enhanced resistance to a pathogenic fungus and/or oomycete" refer to a phenotype in which a plant contacted with strains of the present invention has less severe symptoms, and optionally at least one of greater health, growth, propagation, fertility, vigor, strength (e.g., stem strength and resistance), and yield, associated with infection of the pathogenic fungus or oomycete during or after the fungal or oomycete infection than a plant that was not contacted with the strain. A treated plant with enhanced resistance to a fungal or oomycete pathogen can be infected by the pathogen and exhibit one or more symptoms of infection by the pathogen and yet exhibit a reduction in an effect of the infection or symptom thereof. For instance, a treated plant can be infected by the pathogen, and exhibit one or more symptoms selected from the group consisting of leaf wilt, leaf or vascular discoloration (e.g., yellowing), spike bleaching etc., and yet not exhibit a reduction in yield loss in comparison to a plant that has not been contacted with a strain or strains of the invention.

When a strain of the invention is tested for its capability to confer resistance on a plant or to enhance resistance of a plant, the symptoms associated with the fungal or oomycete infection are compared between a plant or a part thereof treated by contacting the plant or part thereof with strain(s) of the invention and a control plant or part thereof that was not contacted with the strain(s). The control plant is typically, but not necessarily, of the same species as the treated plant. According to some embodiments, the control plant is of the same species and has the same genetic background as the treated plant. The enhancement can be manifested as an increase of 0.1%, 0.2%, 0.3%, 0.5%, 0.75%, 1%, 1.5%, 2%, 3%, 4%, 5%, 6%, 7%, 8%, 9%, 10%, 12%, 15%, 17%, 20%, 25%, 30%, 35%, 40%, 50%, 60%, 70%, 80%, 90%, 100%, or more in health, growth, multiplication, fertility, vigor, strength, or yield, as compared to a control plant. The enhancement can be a decrease of 0.1%, 0.2%, 0.3%, 0.5%, 0.75%, 1%, 1.5%, 2%, 3%, 4%, 5%, 6%, 7%, 8%, 9%, 10%, 12%, 15%, 17%, 20%, 25%, 30%, 35%, 40%, 50%, 60%, 70%, 80%, 90%, or 100% in the symptoms associated with the pathogenic fungus and/or oomycete as compared to the control plant. According to certain exemplary embodiments, the treated plant and the control plant are grown under the same conditions.

Symptoms associated with diseases caused by fungal or oomycete pathogens are known to a person skilled in the art. Typically, a score scale is set for a certain disease based on symptom description and/or severity. Optionally, a specific score scale is set for a combination of a certain disease and a plant species. The use of score scales simplifies the comparison of disease symptoms in plants subjected to various treatments.

According to certain aspects, the present invention provides a method of conferring and/or enhancing resistance to a plant or a part thereof toward a disease caused by a fungus and/or oomycete, comprising contacting the plant or part thereof with at least one bacterial strain or functional homolog thereof according to the present invention, or a preparation of same, or a lysate or extract thereof, or a composition or a formulation comprising same.

According to certain aspects, the present invention provides a method of preventing and/or treating a plant disease caused by a fungus and/or oomycete, comprising contacting the plant or part thereof with at least one bacterial strain or homolog thereof according to the present invention, or a preparation of same, or a lysate or extract thereof, or a composition or a formulation comprising same.

According to certain embodiments, the plant part is selected from the group consisting of a seed, a root, a shoot, a leaf, a branch, a flower, a fruit and any combination thereof. Each possibility represents a separate embodiment of the present invention.

Any method as is known in the art can be used to contact the plant or part thereof with the at least one bacterial strain or functional homolog thereof of the present invention. According to certain embodiments, the plant or part thereof is contacted with a preparation/composition comprising the at least one bacterial strain or functional homolog thereof by a method selected from the group consisting of, but not limited to, immersing, dipping, spraying, coating, dusting and any combination thereof.

According to other embodiments, the plant or part thereof is contacted with the at least one bacterial strain or functional homolog thereof through the plant rhizosphere. According to these embodiments, the preparation/composition is applied to the plant rhizosphere.

According to certain exemplary embodiments, the plant part is a seed and contacting is effected by seed coating.

According to certain exemplary embodiments, the plant part is a leaf and contacting is performed by spraying or dusting.

According to certain exemplary embodiments, the plant part is a root and contacting is performed by dipping or immersing.

According to certain embodiments, the plant or part thereof is surface sterilized prior to contacting with the bacterial preparation/composition/formulation, particularly for research applications.

In some embodiments, the bacterial preparation, compositions and/or formulations comprising same can be contacted with the plant or part thereof, for example the plant seed and/or plant aerial parts, and successful colonization can be confirmed by detecting the presence of the microbial strain within the plant. For example, after applying the composition/formulation/preparation to the seeds, high titers of the microbial strain can be detected in the roots and shoots of the plants that germinate from the seeds. In addition, significant quantities of the microbial strain can be detected in the rhizosphere of the plants. Therefore, in some embodiments, the microbial strain is applied in an amount effective to colonize the plant. The microbial strain may be colonized on the surface of the plant or within the plant tissues as an endophyte. In some embodiments, colonization of the plant can be detected, for example, by detecting the presence of the microbial strain inside the plant. This can be accomplished by measuring the viability of the microbial strain after surface sterilization of the plant portion: microbial strain colonization results in an internal localization of the microbe, rendering it resistant to conditions of surface sterilization. According to some embodiments, the microbial strain is applied in an amount effective to colonize the plant rhizosphere. The presence and quantity of the microbial strain can also be established using other means known in the art, for example, immunofluorescence microscopy using microbe-specific antibodies, or fluorescence of *in situ* hybridization. Alternatively, specific nucleic acid probes recognizing conserved sequences from the colonized bacterial strain can be employed to amplify a region, for example by quantitative PCR, and correlated to CFUs by means of a standard curve.

According to certain exemplary embodiments, the bacterial strain can be detectable within a target tissue of the mature cultivated plant selected from a fruit, a seed, a leaf, or a root, a portion thereof and a combination thereof. It is to be explicitly understood that the bacterial strain or the functional homolog can colonize a plant part distinct from the plant part with which said bacterial strain or functional homolog was contacted

According to certain embodiments, the bacterial strain contacted with the plant, part thereof or the plant rhizosphere is not detected in said plant, plant part thereof and rhizosphere prior to application of said bacterial strain. According to other embodiments, the bacterial strain naturally present in the plant, part thereof, and/or plant rhizosphere. In any case, the amount of the bacterial strain in the plant, part thereof or plant rhizosphere is higher after contacting with the strain compared to its amount prior to contacting (application).

According to other embodiments, the bacterial strain is applied (contacted), for example, on the surface of at least part of a cultivated plant, in an amount effective in conferring and/or enhancing resistance of the cultivated plant to at least one pathogenic fungus and/or oomycete and/or treating and/or preventing a disease caused by at least one pathogenic fungus and/or oomycete.

According to certain exemplary embodiments, the effective amount is of from about 10⁵-10⁸ CFU.

According to certain embodiments, the fungus and/or oomycete is selected from, but not limited to, *Plasmopara viticola; Erysiphe necator; Fusarium verticillioides; Fusarium graminearum; Botrytis cinerea; Phytophthora capsici; Plasmopora halstedii; Collotetrichum graminicola; Fusarium avenaceum; Fusarium culmorum; Fusarium oxysporum; Fusarium roseum; Fusarium semitectum; Fusarium solani; Fusarium verticillioides; Fusarium verticillioides var. subglutinans; Acremonium strictum; Albugo candida; Albugo tragopogonis; Alternaria alternate; Alternaria brassicae; Alternaria helianthi; Alternaria zinnia; Aphanomyces euteiches; Ascochyta sorghina; Ascochyta tritici; Aspergillus flavus; Bipolaris maydis O; Bipolaris sorghicola; Bipolaris sorokiniana; Cephalosporium acremonium; Cephalosporium gramineum; Cephalosporium maydis; Cercospora kikuchii; Cercospora medicaginis; Cercospora sojina; Cercospora sorghi; Cladosporium herbarum; Clavibacter michiganense subsp. Nebraskense; Clavibacter michiganese subsp. Insidiosum; Claviceps purpurea; Claviceps sorghi; Cochliobolus heterostrophus; Colletotrichum dematium (Colletotichum truncatum); Colletotrichum trifolii; Colletotrichum sublineolum; Corn stunt spiroplasma; Corynespora cassiicola; Curvularia inaequalis; Curvularia lunata; Curvularia pallescens; Diaporthe phaseolorum var. caulivora; Diaporthe phaseolorum var. sojae (Phomopsis sojae); Diplodia macrospora; Erwinia carotovora; Erwinia carotovorum pv. Carotovora; Erwinia chrysanthemi pv. Zea; Erwinia stewartii; Erysiphe cichoracearum; Erysiphe graminis f.sp. tritici; Exserohilum turcicum I, II* & *III; Gaeumannomyces graminis var. tritici; Gibberella zeae (Fusarium graminearum); Gloeocercospora sorghi; Glomerella glycines; Helminthosporium carbonum I, II* & *III (Cochliobolus carbonum); Helminthosporium pedicellatum; Helminthosporium sorghicola; Kabatiella maydis; Leptosphaeria maculans; Leptosphaerulina briosiana; Leptotrichila medicaginis; Macrophomina phaseolina; Microsphaera diffusa; Mycosphaerella brassicicola; Nigrospora oryzae; Penicillium oxalicum; Perconia circinata; Peronosclerospora maydis; Peronosclerospora philippinensis; Peronosclerospora sacchari; Peronosclerospora sorghi; Peronospora manshurica; Peronospora parasitica; Peronospora trifoliorum; Phakopsora pachyrhizi; Phialophora gregata; Phoma insidiosa; Phoma macdonaldii; Phoma medicaginis var. medicaginis; Phomopsis helianthi; Phyllachara sacchari; Phyllosticta maydis; Phyllosticta sojicola; Physoderma maydis; Physopella zeae; Phytophthora cryptogea; Phytophthora megasperma; Phytophthora megasperma f. sp. Glycinea; Pseudocercosporella herpotrichoides; Pseudopeziza medicaginis; Puccinia graminis f.sp. tritici; Puccinia helianthi; Puccinia polysora; Puccinia purpurea; Puccinia recondita f.sp. tritici; Puccinia sorghi; Puccinia striiformis; Pyrenophora tritici-repentis; Pythium aphanidermatum; Pythium arrhenomanes; Pythium debaryanum; Pythium gramicola; Pythium graminicola; Pythium irregular; Pythium splendens; Pythium ultimum; Ramulispora sorghi; Ramulispora sorghicola; Rhizoctonia cerealis; Rhizoctonia solani; Rhizopus arrhizus; Rhizopus oryzae; Rhizopus stolonifera; Sclerophthona macrospora; Sclerospora graminicola; Sclerotinia sclerotiorum; Sclerotinia trifoliorum; Sclerotium rolfsii; Septoria avenae; Septoria glycines; Septoria helianthi; Septoria nodorum; Septoria tritici; Exserohilum turcicum; Sphacelotheca cruenta; Sporisorium reilianum (Sphacelotheca reiliana); Sporisorium sorghi; Stagonospora meliloti; Stemphylium alfalfa; Stemphylium botryosum; Stemphylium herbarum; Stenocarpella maydi (Diplodia maydis); Tilletia indica; Tilletia laevis; Tilletia tritici; Trichoderma viride; Urocystis agropyri; Uromyces striatus; Ustilago maydis; Ustilago tritici; Verticillium albo-atrum; Verticillium dahlia; Pseudoperonospora cubensis, and Aspergillus niger.* Each possibility represents a separate embodiment of the present invention.

Specific pathogenic fungi or oomycetes are known to cause dramatic crop loss due to disease symptoms which negatively affect the quality of the crop. For example, *Fusarium verticilloides* and *Fusarium graminearum* cause rot in maize (specifically stalk rot), wheat, sweet pepper, and eggplants and head blight in wheat. *Fusarium oxysporum* causes sudden death syndrome (SDS) in soybeans, yellow spots in sugar beet, Panama disease in banana, and wilt in tomato, sweet pepper, eggplants, potatoes and various plants of the *Cucurbitaceae* family. *Colletotrichum* spp. cause stalk rot in maize, and anthracnose in sugar beet, tomato and sweet pepper. *Botrytis cinerea* causes Gray Mold in tomato, sweet pepper, eggplant, potato, grapes and many other hosts. Rust is caused by *Puccinia spp.* in maize, wheat and sunflower, by *Uromyces* spp. in sunflower and by *Phakopsora* in soybean. *Phytophthora* causes root rot in soybean, late blight in tomato and potato, blight in eggplant and blight fruit rot in sweet pepper. *Mycosphaerella graminicola* causes leaf blotch in wheat. *Mycosphaerella fijiensis* causes black leaf streak disease (*BLSD*; aka black Sigatoka leaf spot) in banana. *Septoria lycopersici* causes leaf spots in tomato. *Verticillium* spp. cause wilt disease in canola, sugar beet, tomato, sweet pepper, eggplant and potato. *Magnaporthe oryza* causes rice blast. *Pythium* spp. cause damping off disease in maize, soybean, tomato, sweet pepper, eggplant and potato and black vessels in sugar beet. *Sclerotinia* causes stem rot in soybean and white mold in tomato, sweet pepper, eggplant and potato. *Rhizoctonia solani* causes root crown rot in sugar beet, sheath blight in rice, and damping-off disease in tomato, sweet pepper, eggplant and potato. Maize smut is caused by *Ustilago maydis. Alternaria* spp. cause leaf spots in sugar beet and sweet pepper, early blight in tomato and potato, and fruit rot in sweet pepper and eggplants. *Cercospora* causes leaf blight in soybean and leaf spots in sugar beet, sweet pepper, eggplants and potato. *Macrophomina* causes charcoal rot in maize, wheat, soybean, tomato and potato. *Sclerotium rolfsii* causes Southern blight in sweet pepper and eggplants. *Oidium spp.* cause powdery mildew in tomato, sweet pepper, eggplants and potato. Powdery mildew is also caused by *Blumeria graminis.*

*Plasmopara viticola* causes lesions on young leaves that become dry and necrotic, and necrosis of young inflorescences and young berries.

*Pseudoperonospora cubensis* causes Downy Mildew in *Cucubirtaceae.* Presence of *Aspergillus niger* on grapes causes Black Mold.

The term "plant" as used herein encompasses a whole plant, a grafted plant, ancestor(s) and progeny of the plants and plant parts, including seeds, shoots, stems, roots (including tubers), rootstock, scion, and plant cells, tissues and organs. The plant or part thereof may be in any form including suspension cultures, embryos, meristematic regions, callus tissue, leaves, gametophytes, sporophytes, pollen, and microspores. Plants to be treated according to the methods of the invention include all plants which belong to the superfamily *Viridiplantae*, in particular monocotyledonous and dicotyledonous plants including a fodder or forage legume, ornamental plant, food crop, tree, or shrub selected from the list comprising *Acacia spp., Acer spp., Actinidia spp., Aesculus spp., Agathis australis, Albizia amara, Alsophila tricolor, Andropogon spp., Arachis spp* (peanut), *Areca catechu, Astelia fragrans, Astragalus cicer, Baikiaea plurijuga, Betula spp., Brassica spp.* (broccoli, Brussels sprouts, collard greens, cabbage, rapeseed/oilseed rape/canola, cauliflower, kale), *Bruguiera gymnorrhiza, Burkea africana, Butea frondosa, Cadaba farinosa, Calliandra spp, Camellia sinensis* (tea), *Canna indica, Capsicum spp.* (pepper), *Cassia spp., Centroema pubescens, Chacoomeles spp., Cinnamomum cassia, Coffea arabica, Colophospermum mopane, Coronillia varia, Cotoneaster serotina, Crataegus spp., Cucumis spp.* (squash), *Cupressus spp., Cyathea dealbata, Cydonia oblonga, Cryptomeria japonica, Cymbopogon spp., Cynthea dealbata, Cydonia oblonga, Dalbergia monetaria, Davallia divaricata, Desmodium spp., Dicksonia squarosa, Dibeteropogon amplectens, Dioclea spp, Dolichos spp., Dorycnium rectum, Echinochloa pyramidalis, Ehraffia spp., Eleusine coracana, Eragrestis spp., Erythrina spp., Eucalyptus spp.* (eucalyptus), *Euclea schimperi, Eulalia vi*/*losa, Pagopyrum spp., Feijoa sellowlana, Fragaria spp.* (strawberry), *Flemingia spp, Freycinetia banksli, Geranium thunbergii, Ginkgo biloba, Glycine max* (soybean), *Gliricidia spp, Gossypium hirsutum* (cotton), *Grevillea spp., Guibourtia coleosperma, Hedysarum spp., Hemaffhia altissima, Heteropogon contoffus, Hordeum vulgare* (barley), *Hyparrhenia rufa, Hypericum erectum, Hypeffhelia dissolute, Indigo incamata, Iris spp., Leptarrhena pyrolifolia, Lespediza spp., Lettuca spp., Leucaena leucocephala, Loudetia simplex, Lotonus bainesli, Lotus spp., Macrotyloma axillare, Malus spp., Manihot esculenta, Medicago sativa* (alfalfa), *Metasequoia glyptostroboides, Musa sapientum, Nicotianum spp.* (tobacco), *Onobrychis spp., Ornithopus spp., Oryza spp.* (rice), *Peltophorum africanum, Pennisetum spp., Persea gratissima, Petunia spp., Phaseolus spp., Phoenix canariensis, Phormium cookianum, Photinia spp., Picea glauca, Pinus spp., Pisum sativum* (pea), *Podocarpus totara, Pogonarthria fleckii, Pogonaffhria squarrosa, Populus spp., Prosopis cineraria, Pseudotsuga menziesii, Pterolobium stellatum, Pyrus communis, Quercus spp., Rhaphiolepsis umbellata, Rhopalostylis sapida, Rhus natalensis, Ribes grossularia, Ribes spp., Robinia pseudoacacia, Rosa spp., Rubus spp., Salix spp., Schyzachyrium sanguineum, Sciadopitys vefficillata, Sequoia sempervirens, Sequoiadendron giganteum, Solanum spp.* (potato, tomato, eggplant), *Sorghum bicolor, Spinacia spp., Sporobolus fimbriatus, Stiburus alopecuroides, Stylosanthos humilis, Tadehagi spp, Taxodium distichum, Themeda triandra, Trifolium spp., Triticum spp.* (wheat), *Tsuga heterophylla, Vaccinium spp., Vicia spp., Vitis vinifera* (grape), *Watsonia pyramidata, Zantedeschia aethiopica, Zea mays* (maize), amaranth, artichoke, asparagus, carrot, celery, flax, lentil, okra, onion, sugar beet, sugar cane, sunflower, rye, oat, sunflower, a tree, an ornamental plant, a perennial grass and a forage crop.

According to certain exemplary embodiments, the pathogenic fungus is selected from the group consisting of *Fusarium graminearum* and *Fusarium verticilloides,* and the bacterial strain is selected from the group consisting of LAV43122, LAV46348, LAV49623, LAV60067, LAV61190, functional homologs thereof and any combination thereof.

According to certain exemplary embodiments, the pathogenic oomycete is *Phytophthora capsici* and the bacterial strain is selected from the group consisting of LAV43122, LAV48853, LAV59924, LAV60064, LAV60067, LAV60072, LAV61190, functional homologs thereof, and any combination thereof.

According to certain exemplary embodiments, the pathogenic oomycete is *Plasmopara viticola,* which causes Downy Mildew disease, and the bacterial strain is selected from the group consisting of LAV43723, LAV61190, functional homologs thereof and any combination thereof. According to certain exemplary embodiments, the plant contacted with the bacterial strain is a grapevine.

According to certain exemplary embodiments, the pathogenic fungus is *Erysiphe necator,* which causes Powdery Mildew disease, and the bacterial strain is selected from the group consisting of LAV49648, LAV43122, LAV54823, LAV48853, LAV60062, LAV60063, LAV60069, LAV60070, LAV60072, functional homologs thereof, and any combination thereof. According to certain exemplary embodiments, the plant contacted with the bacterial strain is a grapevine.

According to certain exemplary embodiments, the pathogenic fungus is *Botrytis cinerea,* which causes Gray Mold disease, and the bacterial strain is selected from the group consisting of LAV43122, LAV54823, LAV49762, LAV49648, LAV49623, LAV34085, LAV46348, LAV59924, LAV60063, LAV60064, LAV60067, LAV60069, LAV60070, LAV60072, LAV60096, functional homologs thereof and any combination thereof. According to certain exemplary embodiments, the plant contacted with the bacterial strain is a tomato plant. According to certain exemplary embodiments, the plant contacted with the bacterial strain is a grapevine.

According to certain exemplary embodiments, the pathogenic fungus is *Aspergillus niger,* which causes Black Mold disease, and the bacterial strain is selected from the group consisting of LAV49762, LAV54823, functional homologs thereof and any combination thereof. According to certain exemplary embodiments, the plant contacted with the bacterial strain is a grapevine.

According to certain exemplary embodiments, the pathogenic fungus is *Fusarium oxysporum,* which causes *Fusarium* seedling blight symptoms, and the bacterial strain is selected from the group consisting of LAV43122, LAV46348, functional homologs thereof and any combination thereof. According to certain exemplary embodiments, the plant contacted with the bacterial strain is a corn plant.

According to certain exemplary embodiments, the pathogenic fungus is selected from the group consisting of *Fusarium oxysporum* and *Fusarium graminearum,* which cause *Fusarium* seedling blight symptoms, and the bacterial strain is selected from the group consisting of LAV43122, LAV46348, LAV49623, LAV60067, LAV61190, functional homologs thereof and any combination thereof. According to certain exemplary embodiments, the plant contacted with the bacterial strain is selected from the group consisting of a tomato plant and a corn (i.e. maize) plant.

According to certain embodiments, the bacterial strain LAV49762 is effective in protecting plants against *Botrytis cinerea,* which causes Gray Mold disease and *Aspergillus niger,* which causes Black Mold disease.

According to certain embodiments, the bacterial strain LAV54823 is effective in protecting plants against *Erysiphe necator,* which causes Powdery Mildew disease; *Aspergillus niger,* which causes Black Mold disease; and *Botrytis cinerea,* which causes Gray Mold disease.

According to certain embodiments, the bacterial strain LAV43122 is effective in protecting plants against *Erysiphe necator,* which causes Powdery Mildew disease; *Botrytis cinerea,* which causes Gray Mold disease; *Fusarium oxysporum, Fusarium graminearum,* and a combination thereof, which cause *Fusarium* seedling blight symptoms; and against disease caused by *Fusarium verticilloides* and *Phytophthora capsici.*

According to certain embodiments, the bacterial strain LAV60072 is effective in protecting plants against *Erysiphe necator,* which causes Powdery Mildew disease; *Botrytis cinerea,* which causes Gray Mold disease; and against disease caused by *Phytophthora capsici.*

According to certain embodiments, the bacterial strain LAV60067 is effective in protecting plants against *Botrytis cinerea,* which causes Gray Mold disease; *Fusarium oxysporum, Fusarium graminearum,* and a combination thereof, which cause *Fusarium* seedling blight symptoms; and against diseases caused by *Phytophthora capsici* and *Fusarium verticilloides.*

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

The following examples are presented in order to more fully illustrate some embodiments of the invention. They should, in no way be construed, however, as limiting the broad scope of the invention. One skilled in the art can readily devise many variations and modifications of the principles disclosed herein without departing from the scope of the invention.

### EXAMPLES

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Patent Nos. 4,683,202;; and 5,192,659; "Cell Biology: A Laboratory Handbook", Volumes I-III Cellis, J. E., ed. (1994); ""Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### Example 1: Sourcing of microbial strains with potential biofungicidal activity

This example describes the source from which the microbial strains were isolated prior to screening for bio-fungicidal activity. Table 1 hereinbelow presents the microbial strains described in this invention and their origin.

### Experimental procedures

Sampling was carried out during the years 2017-2019. Source plants were sampled from various relevant habitats across Israel. Source plant organs (leaves, berries, roots) were removed. In the laboratory, the detached organs were immersed in sterile phosphate buffered saline [PBS; per liter: 8 gr sodium chloride (NaCl), 0.2 gr potassium chloride (KCl), 1.42 gr disodium phosphate (Na2HPO4) and 0.24 gr potassium phosphate (KH2PO4), pH7.4] and shaken for 30 min at 200 revolutions per minute (RPM).

Thereafter, the plant organs were transferred carefully to a new 50 ml Falcon tube. Microbes released from the plant organs into the medium were serially diluted and the selected dilutions were plated onto several bacteriological growth media including, but not limited to, R2G [per liter: 0.5 g proteose peptone, 0.5 g casamino acids, 0.5 g yeast extract, 0.5 g dextrose, 0.5 g soluble starch, 0.3 g dipotassium phosphate (K₂HPO₄), 0.05 g, magnesium sulfate (MgSO₄•7H₂O), 0.3 g sodium pyruvate and 8 g gelrite as a gelling agent]; PDA (per liter: 4 g potato extract, 20 g dextrose and 20 g agar); NA (per liter: 5 g peptone and 15 g agar as gelling agent); NYDA (per liter: 7 g peptone, 5 g yeast extract, 1 g glucose, 4 g NaCl and 13 g agar as gelling agent); Actinomycete Isolation Agar (per liter: 0.1 g L-asparagine. 0.5 g K₂HPO₄, 0.001 g FeSO₄ and 15 g agar as gelling agent).

Isolated colonies that appeared on plates after 24-72 hours of growth at 28°C, in the dark, were further picked and re-isolated on a new R2G plate, before storage in an R2A broth [per liter: 0.5 g proteose peptone, 0.5 g casamino acids, 0.5 g yeast extract, 0.5 g dextrose, 0.5 g soluble starch, 0.3 g dipotassium phosphate (K₂HPO₄), 0.05 g magnesium sulfate (MgSO₄•7H₂O), 0.3 g sodium pyruvate], supplemented with 25% glycerol, at minus 80°C. Isolates were identified to the strain level by whole genome sequencing using an Illumina MiSeq sequencing platform, or to the species level by Sanger sequencing of the 16S-rRNA gene with the universal primers 16S_27F and 16S_1492R (see Example 2 hereinbelow).

**Table 1**

| **Microbial strains described according to some embodiments of the invention** | | | |
|---|---|---|---|
| **Microbial strain name** | **Origin** | **Plant type** | **Plant tissue** |
| LAV34085 | *Hyparrhenia hirta* | Wild plant | Rhizosphere |
| LAV43122 | Soil from Wheat (*Triticum aestivum*) field | NR | NR |
| LAV43723 | Grape *(Vitis vinifera)* | Commercial variety | Phyllosphere |
| LAV46348 | Forest soil | NR | NR |
| LAV48853 | Corn *(Zea mays)* | Commercial variety | Rhizoplane |
| LAV49623 | Muskmelon (*Cucumis melo*) | Commercial variety | Phyllosphere |
| LAV49648 | Common Sunflower (*Helianthus anuus)* | Commercial variety | Phyllosphere |
| LAV49762 | Muskmelon (*Cucumis melo*) | Commercial variety | Phyllosphere |
| LAV54823 | Grape (*Vitis vinifera*) | Commercial variety | Phyllosphere |
| LAV59924 | Wheat (*Triticum aestivum*) | Commercial variety | Rhizosphere |
| LAV60063 | Wheat (*Triticum aestivum*) | Commercial variety | Rhizosphere |
| LAV60064 | Corn *(Zea mays)* | Commercial variety | Seed |
| LAV60067 | Grape *(Vitis vinifera)* | Commercial variety | Carposphere (Berries) |
| LAV60069 | Grape *(Vitis vinifera)* | Commercial variety | Phyllosphere |
| LAV60070 | *Soybean (Glycine max)* | Commercial variety | Carposphere (Stem) |
| LAV60072 | Corn *(Zea mays)* | Commercial variety | Phyllosphere |
| LAV61190 | Grape (*Vitis vinifera*) | Commercial variety | Phyllosphere |

| | | | |
|---|---|---|---|
| NR: Not relevant | | | |

### Example 2: Microbial strain characterization by 16S-rRNA

The microbial strains of the present invention have been further characterized based on their 16S-rRNA sequence. Without wishing to be bound by any theory or mechanism of action, any strain with a significantly homologous 16S-rRNA sequence is likely to exhibit the same functional properties and therefore can be used as a fungicide as described in this invention.

### Experimental procedures

16S-rRNA sequences were obtained by either:
1) Polymerase Chain Reaction (PCR; Mullis, K.B., et al. 1987. Process for amplifying, detecting, and/or-cloning nucleic acid sequences. U.S. Patent 4,683,195) using the universal primers 16S_27F (AGAGTTTGATCMTGGCTCAG, SEQ ID NO:43) and 16S_1492R (TACGGYTACCTTGTTACGACTT, SEQ ID NO:44) (Eden, P.A., et al. 1991. Phylogenetic analysis of Aquaspirillum magnetotacticum using polymerase chain reaction-amplified 16S rRNA-specific DNA. Int. J. Syst. Bacteriol. 41:324-325) followed by Sanger sequencing (Sanger, F., and Coulson, A.R. 1975. A rapid method for determining sequences in DNA by primed synthesis with DNA polymerase. J. Mol. Biol. 94:441-448) of the amplified fragments using the primers 16S_27F and 16S_1492R, and the additional primers 16S_5151F (GTGCCAGCMGCCGCGGTAA, SEQ ID NO:45) and 16S_970R (CCGTCAATTCMTTTRAGTTT, SEQ ID NO:46).
2) Extraction of 16S-rRNA sequences from the assembly of microbial strains genome sequences (Applicant proprietary pipeline) using the MOTHUR tool (a bioinformatics tool for analyzing 16S-rRNA gene sequences; Schloss, P.D., Westcott, S.L., Ryabin, T., Hall, J.R., Hartmann, M., Hollisteret, E.B., al. 2009. Introducing mothur: open-source, platform-independent, community-supported software for describing and comparing microbial communities. Appl. Environ. Microbiol. 75:7537-7541) and SILVA database (a comprehensive on-line resource for quality checked and aligned ribosomal RNA sequence data; Pruesse, E., Quast, C., Knittel, K., Fuchs, B.M., Ludwig, W., Peplies, J., and Glöckner, F.O. 2007. SILVA: a comprehensive online resource for quality checked and aligned ribosomal RNA sequence data compatible with ARB. Nucl. Acids Res. 35:7188-7196) as a reference.

Obtained 16S-RNA sequences were clustered (grouped) to Operational Taxonomic Units (OTUs) using nucleotide-based local alignment search tool (BLASTN; Altschul, S., Gish, W., Miller, W., Myers, E.W., and Lipman, D.J. 1990. Basic local alignment search tool. J. of Mol. Biol. 215:403-410).

### Table 2

**Table 2: List of bacterial strains of the invention, their NCIMB deposit numbers, and their respective 16S-rRNA sequences as disclosed in the Sequence Listing herein. Some 16S-rRNA sequences showed homology higher than 97% global identity to sequences of other strains of the invention, as detailed in the rightmost column.**

| **Bacterial strain organism and 16S rRNA SEQ ID NOs.** | | | | |
|---|---|---|---|---|
| **Isolate ID Number** | **NCIMB Deposit No.** | **Organism** | **16S rRNA SEQ ID NOs.** | **Homologous sequences (over 97%)** |
| LAV34085 | 43434 | *Serratia plymutica* | 1 | |
| LAV43122 | 43431 | *Pseudomonas chlororaphis* | 2-3 | SEQ ID NO:5 (homologous to SEQ ID NO:3) |
| LAV43723 | 43436 | *Streptomyces fulvissimus* | 4 | |
| LAV46348 | 43435 | *Pseudomonas fluorescens* | 5 | SEQ ID NO:3, SEQ ID NO: 13 |
| LAV48853 | 43611 | *Pseudomonas fluorescens* | 6 | SEQ ID NO:13 |
| LAV49623 | 43429 | *Pantoea vagans* | 7 | |
| LAV49648 | 43432 | *Enterobacter cloacae* | 8-9 | SEQ ID NO:18 (homologous to SEQ ID NO:8) |
| LAV49762 | 43430 | *Pseudomonas rhizosphaerae* | 10 | |
| LAV54823 | 43433 | *Erwinia gerundensis* | 11 | |
| LAV59924 | 43610 | *Pantoea agglomerans* | 12 | |
| LAV60063 | 43612 | *Pseudomonas fluorescens* | 13 | SEQ ID NO:5, SEQ ID NO:6 |
| LAV60064 | 43613 | *Pseudomonas psychrotolerans* | 14 | |
| LAV60067 | 43609 | *Gluconobacter frateurii* | 15 | |
| LAV60069 | 43606 | *Bacillus endophyticus* | 16 | |
| LAV60070 | 43608 | *Enterobacter cloacae* | 17-18 | SEQ ID NO:8 (homologous to SEQ ID NO: 18) |
| LAV60072 | 43614 | *Pseudomonas azotoformans* | 19 | |
| LAV61190 | 43607 | *Bacillus velezensis* | 20 | |

### Example 3: Clustering of microbial strains using strain-specific genomic markers

### Experimental procedures

DNA fragments with lengths ranging from 200 bp to 1000 bp from genomes of the microbial strains of certain embodiments of this invention were screened against the NCBI bacteria genome refseq nucleotide database, using NCBI local alignment tool BLASTN (NCBI-blast-2.10.0+). Criteria for declaring a microbial strain-specific marker are at least 90% coverage with at least 95% local sequence identity. 1-5 microbial strain-specific genomic markers were selected for each microbial strain described in this invention.

**Table 3**

| **Genomic Marker SEQ ID NOs. per strain** | | | |
|---|---|---|---|
| **Microbial strain number** | **Genomic Marker SEQ ID NOs.** | **Number of Microbial strain-specific markers** | **Genomic Marker Sequences length (nucleotides), concordant with Marker SEQ ID NOs.*** |
| LAV34085 | 21-25 | 5 | 491;451;449;446;436 |
| LAV43122 | 26-27 | 2 | 320;320 |
| LAV43723 | 28-32 | 5 | 374;365;344;308;289 |
| LAV46348 | 33-37 | 5 | 428;381;354;341;207 |
| LAV48853 | 38-42 | 5 | 569;474;407;392;379 |
| LAV49623 | 43-47 | 5 | 499;487;484;480;480 |
| LAV49648 | 48-52 | 5 | 478;461;454;437;332 |
| LAV49762 | 53-57 | 5 | 489;487;484;477;472 |
| LAV54823 | NA | NA | NA |
| LAV59924 | NA | NA | NA |
| LAV60063 | 58-59 | 2 | 374;212 |
| LAV60064 | 60-61 | 2 | 634;241 |
| LAV60067 | 62-66 | 5 | 413;396;330;289;267 |
| LAV60069 | 67-71 | 5 | 639;606;523;446;245 |
| LAV60070 | NA | NA | NA |
| LAV60072 | 72 | 1 | 306 |
| LAV61190 | NA | NA | NA |

| | | | |
|---|---|---|---|
| Table 3: *NA - Not Available (no genomic marker sequences were found meeting the criteria detailed above). | | | |

### Example 4: Screening and evaluation of potential fungal biocontrol activity of bacterial isolates

Microbial strains obtained as described hereinabove were screened for their ability to suppress the mycelial growth of *Fusarium graminearum*, *Fusarium verticilloides*, *Botrytis cinerea* and *Phytophthora capsici* by *in vitro* dual culture assays on potato dextrose agar (PDA, as described in Example 1 hereinabove).

### Experimental Procedures

Fungal cultures were routinely maintained on PDA medium plates. Isolated microbial strains were grown in R2A medium (as described in Example 1 hereinabove) at 28°C for 48 hours. Cell pellets were then collected by centrifugation at 10,000rpm at room temperature for 5 minutes and re-suspended in 2 ml of sterile PBS (as described in Example 1 hereinabove).

A PDA agar plug from a fungal culture was placed in the center of a PDA Petri dish (bottom-up) and 5 microliters of a bacterial cell culture were plated at 2.5 cm distance from the center. Four different bacteria were included in each plate. Each combination of pathogenic fungus/bacteria was replicated 3 times and plates were randomly placed in the dark and incubated at 25°C for 5 days. As negative controls, 3 Petri dishes were inoculated only with the fungi cultures. The radial growth of the fungus mycelium colony towards a specific bacterial colony was measured (mm) and the average inhibition was calculated relative to the negative control.

Table 4 shows the effects of the microbial strains on the growth of the tested fungal pathogens in the *in vitro* dual culture assays.

### Table 4

**Table 4: The radial mycelial growth of the fungi towards the antagonistic bacteria (Ri) and that on a control plate (Rc) were measured and the mycelial growth inhibition (%) was calculated according to the formula: (Rc-Ri)/Rc × 100. Numbers in parentheses denote p-value (t-test, significant at P≤0.05).**

| ***in vitro* inhibition of fungal growth by the microbial strains** | | | | |
|---|---|---|---|---|
| **Microbial strain** | **Fungal Species** | | | |
| | ***Fusarium graminearum*** | ***Fusarium verticilloides*** | ***Botrytis cinerea*** | ***Phytophthora capsici*** |
| LAV34085 | 6 (0.05) | 4.2 (0.3) | 3 (0.5) | 4 (0.004) |
| LAV43122 | 49 (0.0001) | 42 (0.00) | 17.4 (0.002) | 22 (0.0003) |
| LAV43723 | 0 (0.3) | 0 | 0 (0.07) | 0 |
| LAV46348 | 21 (0.002) | 6.3 (0.00) | 11 (0.005) | 8 (0.007) |
| LAV48853 | 4 (0.1) | 3 (0.25) | 4.5 (0.04) | 10 (0.004) |
| LAV49623 | 15 (0.009) | 8.4 (0.000) | 0.7 (0.1) | 7 (0.01) |
| LAV49648 | 0 (0.3) | 4.2 (0.3) | 3 (0.3) | 0 |
| LAV49762 | 0 (0.3) | 2.8 (0.3) | 0 (0.07) | 0 |
| LAV54823 | 0 (0.3) | 4.2 (0.3) | 2 (0.5) | 0 |
| LAV59924 | 2 (0.17) | 3 (0.27) | 0 (0.04) | 21 (0.001) |

| **Microbial strain** | **Fungal Species** | | | |
|---|---|---|---|---|
| | ***Fusarium graminearum*** | ***Fusarium verticilloides*** | ***Botrytis cinerea*** | ***Phytophthora capsici*** |
| LAV60063 | 0 (0.04) | 0 (0.03) | 0 (0.17) | 0 (0.04) |
| LAV60064 | 2 (0.17) | 3 (0.24) | 0 (0.04) | 17 (0.003) |
| LAV60067 | 27 (0.03) | 32 (0.003) | 9 (0.02) | 36 (0.007) |
| LAV60069 | 1 (0.25) | 0 (0.06) | 0 (0.04) | 0 (0.05) |
| LAV60070 | 0 (0.3) | 4 (0.3) | 2 (0.5) | 5 (0.02) |
| LAV60072 | 4 (0.14) | 6 (0.5) | 0 (0.1) | 12 (0.02) |
| LAV61190 | 20 (0.002) | 21 (0.002) | 23 (0.001) | 28 (0.01) |

### Example 5: In vivo inhibition of Downy Mildew disease development in detached grapevine leaves

### Experimental Procedures

Isolated microbial strains were grown in R2A medium (as described in Example 1 hereinabove) at 28°C for 24 hours. Cell pellets were then collected by centrifugation at 10,000 rpm at room temperature for 5 minutes and re-suspended in 10 ml of sterile PBS. Cell density of each microbial suspension was determined (O.D. 600). Cell concentration was determined by plating serial dilution (in PBS) on R2G plates, counting and calculating Colony Forming Units (CFUs) after 2 days of growth at 28°C in the dark.

Inhibition of Downy Mildew disease caused by *Plasmopara viticola* was evaluated using a detached leaf assay essentially as described by Prajongjai et al. (S. Afr. J. Eno. Vitic 35:43; 2014). Leaves of similar age and area were taken from plants grown in a greenhouse at Evogene Farm (Israel) from two primary grape (*V*. *vinifera)* varieties: Cabernet and Merlot.

The leaves were surface-disinfected with 0.09% (w/v) sodium hypochlorite and 0.01% (v/v) Tween 20, rinsed with sterile distilled water, and placed abaxial surface up on moist filter paper in a Petri dish.

Bacterial suspensions were sprayed on the leaf surface until run-off: about 1 ml suspension/leaf corresponding to a bacterial concentration of 10⁷-10⁸ CFU/ml, (4 leaf repeats per concentration), and allowed to dry before pathogen application.

*Plasmopara viticola* sporangia were collected from Downy Mildew-infected *V*. *vinifera* leaves routinely maintained in a controlled growth chamber by flooding the leaf surface with water. The sporangial suspension concentration was estimated using a hemocytometer and adjusted to 1 × 10⁴ sporangialml.

To examine the anti-fungal activity of the bacteria strains, the sporangial suspensions were sprayed onto the abaxial leaf surface of each detached leaf described above until evenly wet. Leaves with no prior bacterial treatment (fungi only) served as the normal disease development control for the experiment. Petri dishes were held at 24°C, 16 h photoperiod for 7 days. Infected leaves were transferred into an incubator at 19°C at 100% relative humidity (RH) overnight for enhanced sporulation for disease estimation. Disease severity of infected leaf area was classified into 3 classes: 0 = no sporulation detected; 1 = 10-20% infected leaf area; 2 = 30-50% infected leaf area; 3 = >50% infected leaf area. Percentage disease inhibition afforded by the bacterial treatment was calculated relative to the untreated leaves' score.

Table 5 shows the microbial strains that significantly decreased Downy Mildew development in grapevine detached leaves compared to the non-treated control (Fisher Exact test, p-value < 0.2).

### Table 5

**Table 5: Inhibition of Downy Mildew development on detached leaves calculated according to the formula (DSc - DSt)/DSc × 100, wherein DSc-disease score in untreated (control) leaves; DSt - disease score in leaves treated with the indicated microbial strain. Numbers in parentheses denote concentration of bacterial strain applied.**

| **Microbial strains inhibiting Downy Mildew development in detached leaves of grapevine plants** | | |
|---|---|---|
| **Microbial strain number** | **% Inhibition** | **P value** |
| LAV43723 | 100 (10⁸ CFU) | 0.04 |
| LAV61190 | 45 (10⁶ CFU) | 0.05 |

### Example 6: In vivo inhibition of Downy Mildew disease in intact grapevine plants

Bacterial strains LAV43723 and LAV61190, which successfully passed the detached leaf assay, were evaluated for antifungal activity on whole grapevine plants in a greenhouse-controlled environment.

Cuttings were taken from grapevines containing four or five nodes and four or more internodes on each section. A clean cut was made straight across just below a leaf node, and the basal end planted in gardening soil. Young plants with up to two shoots were used for the whole plant assays. Leaves of similar age were chosen, marked and the abaxial side of each leaf was treated with a bacterial suspension followed by pathogen application as described in Example 3 hereinabove. In this experiment the pathogen was applied 24 hours (LAV43723) or 48 hours (LAV61990) after the bacteria application.

The isolate was tested on 5 plants. Two leaves on each plant were treated, one with a bacterial suspension at 10⁷ CFU/ml and the other at 10⁸ CFU/ml.

After infection, the plants were kept overnight in a mist chamber at 19°C, 100% RH at the dark and then transferred to the greenhouse at 22-24°C, 50-60% RH, 16 hours light.

After 7 days, treated leaves were cut and placed on moist filter paper in a Petri dish and transferred into an incubator at 19°C at 100% RH overnight for enhanced sporulation. Disease was estimated as described for the detached leaf assay (Example 5 hereinabove). Percentage disease inhibition afforded by the bacterial treatment was calculated relative to the untreated leaves' score.

Table 6 shows significantly decreased Downy Mildew development in treated leaves of intact grapevine plants compared to the non-treated control. (Fisher Exact test, p-value < 0.2)

### Table 6

**Table 6: Inhibition of Downy Mildew development on leaves of intact grapevine plants calculated according to the formula (DSc - DSt)/DSc × 100, wherein DSc-disease score in untreated (control) leaves; DSt - disease score in leaves treated with the indicated microbial strain. Numbers in parentheses denote concentration of bacterial strain applied.**

| **Microbial strains inhibiting Downy Mildew disease development in leaves of intact grapevine plants** | | |
|---|---|---|
| **Microbial strain number** | **% Inhibition** | **P value** |
| LAV43723 | 73 (10⁸ CFU) | 0.007 |

| **Microbial strain number** | **% Inhibition** | **P value** |
|---|---|---|
| LAV61190 | 100 (10⁷ CFU) | 0.001 |

### Example 7: In vivo inhibition of Powdery Mildew disease development in detached grapevine leaves

### Experimental Procedures

The ability of microbial strains of the invention to decrease Powdery Mildew disease development caused by the fungus *Erysiphe necator* was examined using the detached leaf assay, prepared as described in Example 5 hereinabove. Table 7 shows the microbial strains that significantly decreased Powdery Mildew development in grapevine detached leaves compared to the non-treated control (Fisher Exact test, p-value < 0.2).

### Table 7

**Table 7: Inhibition of Powdery Mildew development on detached leaves calculated according to the formula (DSc - DSt)/DSc × 100, wherein DSc-disease score in untreated (control) leaves; DSt - disease score in leaves treated with the indicated microbial strain.**

| **Microbial strains inhibiting Powdery Mildew development in detached leaves of grapevine plants** | | |
|---|---|---|
| **Microbial strain number** | **% Inhibition at 10⁸ CFU** | **P value** |
| LAV43122 | 67 | 0.001 |
| LAV48853 | 72 | 0.001 |
| LAV49648 | 50 | 0.001 |
| LAV54823 | 50 | 0.001 |
| LAV60062 | 55 | 0.05 |
| LAV60063 | 67 | 0.001 |
| LAV60069 | 55 | 0.05 |
| LAV60070 | 61 | 0.001 |
| LAV60072 | 72 | 0.001 |

### Example 8: In vivo inhibition of Powdery Mildew disease in intact grapevine plants

Grapevine plants were prepared from young cuttings as described in Example 6 hereinabove. Two grapevine leaves per plant were treated with a bacterial suspension (10⁸ CFU/ml) or autoclaved bacterial culture, and plants were placed randomly between grapevine plants infected with Powdery Mildew (inoculum source). Each treatment included five plants (10 leaves).

After ten days, the treated leaves were evaluated for presence of mycelium on the upper leaf side and scored on a scale from 0 (no symptoms), 1-10% diseased area, 2-20% diseased area, etc., up to 10 (100% diseased leaf area). Percentage disease inhibition afforded by the bacterial treatment was calculated relative to the untreated leaves' score.

Table 8 shows significant decrease in the development of Powdery Mildew disease in leaves of intact grapevine plants treated with the indicated microbial strains or autoclaved strains compared to the non-treated control (Mann-Whitney one-tailed test, p<0.01).

### Table 8

**Table 8: Inhibition of Powdery Mildew development on leaves of intact grapevine plants calculated according to the formula (DSc - DSt)/DSc × 100, wherein DSc-disease score in untreated (control) leaves; DSt - disease score in leaves treated with the indicated microbial strain.**

| **Microbial strains inhibiting Powdery Mildew development in leaves of intact grapevine plants grown in greenhouse** | | |
|---|---|---|
| **Microbial strain number** | **% Inhibition at 10⁸ CFU** | **P value** |
| LAV43122 | 100 | 0.001 |
| LAV43122 autoclaved | 100 | 0.001 |
| LAV48853 | 100 | 0.001 |
| LAV60063 | 87 | 0.002 |
| LAV60069 | 75 | 0.001 |
| LAV60070 | 87 | 0.007 |

### Example 9: Inhibition of Gray Mold disease development in tomato and grape berries

Tomato or grape berries from different varieties, with uniform appearance and without physical injuries, were washed in a detergent solution, rinsed with sterile tap water and air-dried. The fruit were wounded (a crosscut approximately 4 mm in width and depth) with a sterile knife prior to inoculation with the pathogen.

Five microliters of a spore suspension (5 × 10⁶/ml in 15% glycerol, stored at -80°C) of *Botrytis cinerea,* which causes Gray Mold disease, were inoculated on PDA plates and cultured for 1 week at 22°C. Fungal spores were harvested by flooding the surface of the culture with PBS solution containing 0.05% (v/v) Tween-80, collecting the formed spore suspension, and then filtering the suspension through four layers of sterile cheesecloth. The concentration of the spores in the suspension was determined by observation under optical microscope using a hemocytometer and then adjusting the concentration to 1× 10⁴ per milliliter in PBS.

Wounded berries were sprayed with bacterial suspensions prepared as described in Example 3 hereinabove at 10⁸ and 10⁷ CFU/ml, or with dried isolate culture supernatant. The berries were then air-dried for 24 hours prior to application of the fungal spore solution by spraying the solution on the wounded site. Controls included wounded fruit sprayed with fungal spore suspension only or with the mock solution (PBS).

Inoculated berries were transferred to transparent plastic boxes. About 5ml sterile water was sprayed inside the box to maintain a high relative humidity, and the boxes were stored at 25°C. After 6 days, disease development at the wounded sites was estimated using a visual index (0 = no symptoms 1= minor fungal development 2= medium fungal development, 3= high fungal development). Tables 9 and 10 show microbial strains that significantly decreased the development of Gray Mold disease in tomato and grape berries compared to the non-treated control (Fisher Exact test, p-value < 0.2).

### Table 9

**Table 9: Inhibition of Gray Mold disease caused by Botrytis cinerea in tomato berries, calculated according to the formula (DSc - DSt)/DSc × 100, wherein DSc-disease score in untreated (control) leaves; DSt - disease score in leaves treated with the indicated microbial strain.**

| **Strains inhibiting Gray Mold disease development in tomato berries** | | |
|---|---|---|
| **Microbial strain number** | **% Inhibition at 10⁸ CFU** | **P value** |
| LAV34085 | 73% | 0.08 |

| **Microbial strain number** | **% Inhibition at 10⁸ CFU** | **P value** |
|---|---|---|
| LAV43122 | 60% | 0.05 |
| LAV43122 dried supernatant culture medium | 55% | 0.02 |
| LAV49623 | 67% | 0.025 |
| LAV49648 | 27% | 0.16 |
| LAV49762 | 73% | 0.025 |
| LAV54823 | 87% | 0.009 |
| LAV59924 | 100% | 0.007 |
| LAV60067 | 100% | 0.007 |
| LAV60069 | 100% | 0.007 |

### Table 10

**Table 10: Inhibition of Gray Mold disease caused by Botrytis cinerea in grape berries, calculated according to the formula (DSc - DSt)/DSc × 100, wherein DSc-disease score in untreated (control) leaves; DSt - disease score in leaves treated with the indicated microbial strain.**

| **Strains inhibiting Gray Mold disease development in grape berries** | | |
|---|---|---|
| **Microbial strain number** | **% Inhibition at 10⁸ CFU** | **P value** |
| LAV48853 | 63% | 0.02 |
| LAV59924 | 71% | 0.007 |
| LAV60063 | 72% | 0.01 |
| LAV60064 | 74% | 0.01 |
| LAV60067 | 100% | 0.007 |
| LAV60069 | 75% | 0.01 |
| LAV60070 | 74% | 0.01 |
| LAV60072 | 62% | 0.02 |
| LAV49762 | 45% | 0.1 |
| LAV43122 | 42% | 0.15 |
| LAV43122 dried supernatant culture medium | 86% | 0.02 |

### Example 10: Inhibition of Fusarium seedling blight disease in tomato and corn seedlings

Bacterial isolates were evaluated in a greenhouse assay for the strain capability to reduce *Fusarium* seedling blight symptoms caused by *Fusarium oxysporum* Fol-1 in tomato, or by *Fusarium graminearum* in corn seedlings.

### Tomato seedling assay:

Isolated microbial strains were grown as a lawn on R2A plates for 2 days at 28°C in the dark. Cells were then scraped off the plates and suspended in 20 ml of tap water.

The pathogen (*Fusarium oxysporum* Fol-1) was grown for 8 days in liquid Cz-B medium [Czapek Dox Broth (Sigma 70185) - 10 g/liter; chloramphenicol - 0.075 g/liter] at 25°C with shaking at 150 rpm. The fungal biomass was then washed, re-suspended in water and grinded in a blender. The concentration of the mycelial fraction was then adjusted to 1×10⁶/ml.

For the assay, tomato seeds were sown on sterile vermiculite for 10 days. The germinated seedlings were then pulled out, the root tips were cut (~ 1 cm) and the cut roots were immersed in 10ml of inoculum suspension of *Fusarium oxysporum* containing or not containing (control) the bacterial isolate suspension (10⁹ CFU/ml) for 5 minutes.

Infected seedlings were replanted in the vermiculite, grown in a controlled environment at 26°C and analyzed for disease symptom appearance after 3 weeks.

After 3 weeks, a vertical section was performed along the shoots for disease scoring by evaluating browning of the vascular system indicating mycelial spreading. Disease scores were 0= no symptoms to 5 = browning spread all along the cut.

### Corn seedling assay:

Isolated microbial strains and the *Fusarium graminearum* strain were grown as described for *Fusarium oxysporum* hereinabove.

In the greenhouse, seeds of a commercial maize hybrid were placed in finger-made holes in each pot and 1 ml of bacterial cell suspension (~10⁹ CFU/ ml) was dispensed on top of each seed. Each strain was tested in 5 pot replicates (n = 5; 4 plants per pot). 1 hour later, 1 ml of *Fusarium graminearum* spore suspension (~10⁵ CFU/ ml), was dispensed on top of each seed. After seedling emergence, germination count was documented. Plants were grown under normal conditions until day 21. At the end of the experiment, each plot was washed and disease score documented (score 0 = no symptoms; 1= sporadic root browning; 2= limited browning on root and shoot; 3= extended browning on root; 4= extended browning on root and shoot; 5 = severe browning on root, shoot and seed). In addition, shoot fresh weight was measured. Tables 11 and 12 show microbial strains that significantly decreased Fusarium seedling blight development in corn and tomato, respectively, compared to the non-treated control (Fisher Exact test, p-value < 0.2).

### Table 11

**Table 11: Decrease in symptoms of Fusarium seedling blight disease caused by Fusarium graminearum in corn plants, calculated according to the formula (DSc - DSt)/DSc × 100, wherein DSc-disease score in untreated (control) leaves; DSt - disease score in leaves treated with the indicated microbial strain.**

| **Microbial strains that significantly decrease *Fusarium* seedling blight development in corn** | | |
|---|---|---|
| **Microbial strain number** | **% Inhibition** | **P value** |
| LAV43122 | 50 | 0.25 |
| LAV46348 | 100 | 0.013 |

### Table 12

**Table 12: Decrease in symptoms of Fusarium seedling blight disease caused by Fusarium graminearum in tomato plants, calculated according to the formula (DSc - DSt)/DSc × 100, wherein DSc-disease score in untreated (control) leaves; DSt - disease score in leaves treated with the indicated microbial strain.**

| **Microbial strains that significantly decrease *Fusarium* seedling blight development in tomato** | | |
|---|---|---|
| **Microbial strain number** | **% Inhibition** | **P value** |
| LAV43122 | 50 | 0.2 |
| LAV46348 | 90 | 0.013 |

### Example 11: Vineyard assay for evaluating inhibition of Gray Mold and Black Mold disease

Candidate isolates that passed the berries assay multiple times and on multiple varieties were tested in a field trial in productive vineyards with natural infection by *Botrytis cinerea* or *Aspergillus niger.* Plant culturing conditions (soil type and fertilization) were uniform for all plots and conformed to local viticultural practices. The design and layout of the trials were according to EPPO Standard PP 1/17(2) (Conduct and reporting of efficacy evaluation trials).

For testing Gray Mold development, 10 plants/plot/treatment with 4 repeats were tested. The bacterial pellet used for the treatments was dried using a fluid bed dryer and was applied to the berries at a concentration of 5g/l, corresponding to 10⁷-10⁸ CFU/ml, by a manual sprayer 5 times at 10-day intervals, from early maturation until harvest. After 7-10 days, disease was characterized on grape bunches (50 bunches per vine) and compared to untreated control plants and to commercially available biological (*Bacillus amyloliquefaciens*) and chemical (Cyprodinil/Fludioxonil) agents as positive controls.

For testing Black Mold development, 10 bunches/plot/treatment with 4 repeats (total 40 bunches/treatment) were marked prior to bacterial application. Bacterial isolates were applied to berries as in the Gray Mold assay above. After 10 days, disease was characterized on bunches and compared to untreated control plants and to a commercially available chemical agent (Pyrimethanil) as a positive control.

Tables 13 and 14 show comparisons of the disease incidence and severity in treated berries to those of the controls.

### Table 13

**Table 13: Comparison of Gray Mold disease incidence and severity in grape berries treated with microbial strains of the invention (LAV43122 and LAV49762) as compared with commercially available agents (biological and chemical) as positive controls and an untreated negative control. a,b,c denote statistical grouping according to Levene' s F Test LSD, p value<0.05.**

| **Disease incidence and Gray Mold severity assessment at harvest** | | |
|---|---|---|
| **Treatment** | **Incidence %** | **Severity %** |
| LAV43122 | 30^{b} | 7^{c} |
| LAV49762 | 28^{b} | 6^{c} |
| Biological positive control | 43.5^{a} | 11^{b} |
| Chemical positive control | 24.5^{b} | 4^{c} |
| Untreated control | 48.5^{a} | 14.6^{a} |

### Table 14

**Table 14: Comparison of Black Mold disease incidence and severity in grape berries treated with microbial strains of the invention (LAV49762 and LAV54823) as compared with a commercially available chemical agent as a positive control and an untreated negative control. a,b,c denote statistical grouping according_to ANOVA Test, p value<0.05.**

| **Disease incidence and Black Mold severity assessment at harvest** | | |
|---|---|---|
| **Treatment** | **Incidence %** | **Severity %** |
| LAV49762 | 47.6^{abc} | 0.8^{b} |
| LAV54823 | 37.2^{bc} | 0.6^{bc} |
| Chemical positive control | 25.7^{c} | 0.4^{c} |
| Untreated control | 67.5^{a} | 1.3^{a} |

### Example 12: Inhibition of Pythium seedling wilt in a corn seedling assay

*Pythium aphanidermatum* was grown on PDA plates for 48h at 25°C. 1 cm diameter discs were cut from the culture edge to inoculate 100g autoclaved pearl millet seeds with the addition of 30 ml sterile water. The inoculated seeds were incubated at 25°C for 6 days and ground to a fine mixture. 1g of the mixture was used to inoculate 200g of planting soil mixture [Peat (Kekila) and perlite (1:2, v/v)]. Corn seeds (Supersweet) were then planted in the mixture in trays. Each one of two repeat experiments included 24 seeds per treatment. 1ml of Bacteria solution (10⁸ CFU/ml) was added at planting time. One ml of sterile water was added to corn seeds for the mock treatment (untreated control).

After 10 days from planting, seedling emergence was recorded and the percentage was calculated, and compared to that of the untreated control. As is shown in Table 15, 91% of the treated seeds were germinated compared to only 45% germination in the untreated control, thus showing that the examined strain is highly effective in decreasing Pythium seedling wilt.

### Table 15

**Table 15: Inhibition of Pythium seedling wilt in corn seedlings. The value in brackets shows the germination percentage in the untreated control (fungi only). P-value was calculated using Dunnet's test.**

| Microbial **strains that significantly decrease Pythium seedling wilt in corn** | | |
|---|---|---|
| **Microbial strain number** | **% Germination** | **P value** |
| LAV43122 | 91 (45) | 0.0187 |

### Example 13: Function-based clustering of microbial strains

Reduction of plant disease symptoms by application of a microbial strain according to certain embodiments of the present invention is indicative of specific functional properties of the microbial strains. These functional properties contribute to plant tolerance against a disease caused either by a fungus or an oomycete when the microbial strains are present in/on the plant. Microorganisms are known to produce lytic enzymes, especially chitinases, alpha- and beta glucanases and xylanases (CHIs), which hydrolyze chitin, a major component of fungal cell walls, and/or cellulose, the major component of oomycete cell walls. Bacteria producing lytic enzymes are therefore an alternative strategy for controlling phytopathogens. Such bacteria may be clustered according to their lytic properties.

### Xylanase activity

To detect xylanase production ability, 10 microliters of an overnight culture of each one of the microbial strains listed in Table 15 hereinbelow were spotted in 8 replicates on 0.1% xylan agar medium (Composition: g/L; yeast extract 3.0, peptone 1.5, NaCl 3.5, NaNO₃ 1.0, KH₂PO₄ 1.0, MgSO₄·7H₂O 0.3, Agar 20, and 0.1 % beechwood xylan) plates (pH 5.5). Plates were incubated at 28°C ± 2 for 72 h. All the plates were stained with 0.5% Congo red dye for about half an hour and were then de-stained using 1 M NaCl solution at room temperature. Xylanase activity was indicated by zones of clearance scored by visual estimate. (Bushra K & Nazish MA 2016, Optimization of fermentation media and growth conditions for microbial xylanase production. 3 Biotech 6:122).

### Cellulolytic activity

For screening of bacteria producing cellulolytic enzymes, single colonies were inoculated onto Mandels and Reese medium (Mandels M, Reese ET. Induction of cellulase in Trichoderma viride as influenced by carbon sources and metals. Journal of Bacteriology. 1957;73 (2):269-278) containing carboxymethyl cellulose sodium salt (CMC-Na; in g/L: KH₂PO₄, 2.0; (NH₄)₂SO₄, 1.4; MgSO₄ ·7H₂O, 0.3; CaCl₂, 0.3; yeast extract, 0.4; FeSO₄ ·7H₂O, 0.005; MnSO₄, 0.0016; ZnCl₂, 0.0017; CoCl₂, 0.002; CMC-Na, 5.0; and agar, 15.0; pH 5.0). After incubation at 28°C for 48 h, all plates were stained with 1% (w/v) Congo-red solution for 15 min and discolored with 1 M NaCl for 15 min. Degradation zones were visible around the bacteria, showing hydrolyzation of CMC by the inoculated strains.

Table 16 summarizes the results of the Xylanase and Cellulase activities of the microbial strains, as determined from the degradation zones on the assay plates.

### Table 16

**Table 16: Scoring of strains of the invention for Xylanase and Cellulase activity. (-) No activity; (+) clear zone approximately 1mm wide; (++) clear zone approximately 3 mm wide; (+++) clear zone > 3mm wide.**

| **Xylanase and Cellulolytic activity of the microbial strains** | | |
|---|---|---|
| **Microbial strain number** | **Xylanase activity** | **Cellulolytic activity** |
| LAV34085 | - | - |
| LAV43122 | - | - |
| LAV43723 | - | +++ |
| LAV46348 | - | - |
| LAV49623 | + | - |
| LAV49648 | + | +++ |
| LAV49762 | + | - |
| LAV54823 | ++ | + |
| LAV59924 | ++ | + |
| LAV60063 | - | + |
| LAV60064 | - | - |
| LAV60067 | - | - |
| LAV60069 | - | + |
| LAV60070 | - | - |
| LAV60072 | - | + |
| LAV48853 | + | ++ |
| LAV61190 | ++ | + |

### Chitinolytic activity,

For screening of chitinase producing bacteria, an agar solidified medium amended with colloidal chitin is used. The medium consists of (g/ L): Na₂HPO₄, 6; KH₂PO₄, 3; NH₄Cl, 1; NaCl, 0.5; yeast extract, 0.05; agar, 15; and colloidal chitin 1% (w/v). Colonies showing clearance zones on a cream-colored background are considered chitinase-producing bacteria.

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed functions may take a variety of alternative forms without departing from the invention.

### ASPECTS OF THE INVENTION

1. An isolated bacterial strain or a functional homolog thereof, wherein the isolated bacterial strain is selected from the group consisting of:
   (1) strain LAV49762, the strain being selected from the group consisting of
      a. a strain deposited under Accession Number 43430 at NCIMB;
      b. a strain comprising a 16S-rRNA sequence comprising the nucleic acid sequence set forth in SEQ ID NO: 10;
      c. a strain comprising at least one genomic marker comprising the nucleic acid sequence set forth in any one of SEQ ID NOs:53-57; and any combination thereof;
   (2) strain LAV34085, the strain being selected from the group consisting of
      a. a strain deposited under Accession Number 43434 at NCIMB;
      b. a strain comprising a 16S-rRNA sequence comprising the nucleic acid sequence set forth in SEQ ID NO:1;
      c. a strain comprising at least one genomic marker comprising the nucleic acid sequence set forth in any one of SEQ ID NOs:21-25; and any combination thereof;
   (3) strain LAV43122, the strain being selected from the group consisting of
      a. a strain deposited under Accession Number 43431 at NCIMB;
      b. a strain comprising at least one 16S-rRNA sequence comprising the nucleic acid sequence selected from the group consisting of SEQ ID NO:2 and SEQ ID NO:3;
      c. a strain comprising at least one genomic marker comprising the nucleic acid sequence set forth in any one of SEQ ID NOs:26-27; and any combination thereof;
   (4) strain LAV43723, the strain being selected from the group consisting of
      a. a strain deposited under Accession Number 43436 at NCIMB;
      b. a strain comprising a 16S-rRNA sequence comprising the nucleic acid sequence set forth in SEQ ID NO:4;
      c. a strain comprising at least one genomic marker comprising the nucleic acid sequence set forth in any one of SEQ ID NOs:28-32; and any combination thereof;
   (5) strain LAV46348, the strain being selected from the group consisting of
      a. a strain deposited under Accession Number 43435 at NCIMB;
      b. a strain comprising a 16S-rRNA sequence comprising the nucleic acid sequence set forth in SEQ ID NO:5;
      c. a strain comprising at least one genomic marker comprising the nucleic acid sequence set forth in any one of SEQ ID NOs:33-37; and any combination thereof;
   (6) strain LAV49623, the strain being selected from the group consisting of
      a. a strain deposited under Accession Number 43429 at NCIMB;
      b. a strain comprising a 16S-rRNA sequence comprising the nucleic acid sequence set forth in SEQ ID NO:7;
      c. a strain comprising at least one genomic marker comprising the nucleic acid sequence set forth in any one of SEQ ID NOs:43-47; and any combination thereof;
   (7) strain LAV49648, the strain being selected from the group consisting of
      a. a strain deposited under Accession Number 43432 at NCIMB;
      b. a strain comprising at least one 16S-rRNA sequence comprising the nucleic acid sequence selected from the group consisting of SEQ ID NO:8 and SEQ ID NO:9; and
      c. a strain comprising at least one genomic marker comprising the nucleic acid sequence set forth in any one of SEQ ID NOs:48-52; and any combination thereof;
   (8) strain LAV54823, the strain being selected from the group consisting of
      a. a strain deposited under Accession Number 43433 at NCIMB; and
      b. a strain comprising a 16S-rRNA sequence comprising the nucleic acid sequence set forth in SEQ ID NO: 11; and a combination thereof;
   (9) strain LAV60069, the strain being selected from the group consisting of
      a. a strain deposited under Accession Number 43606 at NCIMB;
      b. a strain comprising a 16S-rRNA sequence comprising the nucleic acid sequence set forth in SEQ ID NO: 16;
      c. a strain comprising at least one genomic marker comprising the nucleic acid sequence set forth in any one of SEQ ID NOs:67-71; and any combination thereof; (10) strain LAV61190, the strain being selected from the group consisting of
         a. a strain deposited under Accession Number 43607 at NCIMB;
         b. a strain comprising a 16S-rRNA sequence comprising the nucleic acid sequence set forth in SEQ ID NO:20; and a combination thereof;
   (11) strain LAV60070, the strain being selected from the group consisting of
      a. a strain deposited under Accession Number 43608 at NCIMB; b. a strain comprising at least one 16S-rRNA sequence comprising the nucleic acid sequence selected from the group consisting of SEQ ID NO: 17 and SEQ ID NO: 18; and a combination thereof;
   (12) strain LAV60067, the strain being selected from the group consisting of
      a. a strain deposited under Accession Number 43609 at NCIMB;
      b. a strain comprising a 16S-rRNA sequence comprising the nucleic acid sequence set forth in SEQ ID NO: 15;
      c. a strain comprising at least one genomic marker comprising the nucleic acid sequence set forth in any one of SEQ ID NOs:62-66; and any combination thereof;
   (13) strain LAV59924, the strain being selected from the group consisting of
      a. a strain deposited under Accession Number 43610 at NCIMB;
      b. a strain comprising a 16S-rRNA sequence comprising the nucleic acid sequence set forth in SEQ ID NO: 12; and a combination thereof;
   (14) strain LAV48853, the strain being selected from the group consisting of
      a. a strain deposited under Accession Number 43611 at NCIMB;
      b. a strain comprising a 16S-rRNA sequence comprising the nucleic acid sequence set forth in SEQ ID NO:6;
      c. a strain comprising at least one genomic marker comprising the nucleic acid sequence set forth in any one of SEQ ID NOs:38-42; and any combination thereof;
   (15) strain LAV60063, the strain being selected from the group consisting of
      a. a strain deposited under Accession Number 43612 at NCIMB;
      b. a strain comprising a 16S-rRNA sequence comprising the nucleic acid sequence set forth in SEQ ID NO: 13;
      c. a strain comprising at least one genomic marker comprising the nucleic acid sequence set forth in any one of SEQ ID NOs:58-59; and any combination thereof;
   (16) strain LAV60064, the strain being selected from the group consisting of
      a. a strain deposited under Accession Number 43613 at NCIMB;
      b. a strain comprising a 16S-rRNA sequence comprising the nucleic acid sequence set forth in SEQ ID NO: 14;
      c. a strain comprising at least one genomic marker comprising the nucleic acid sequence set forth in any one of SEQ ID NOs:60-61; and any combination thereof; and
   (17) strain LAV60072, the strain being selected from the group consisting of
      a. a strain deposited under Accession Number 43614 at NCIMB;
      b. a strain comprising a 16S-rRNA sequence comprising the nucleic acid sequence set forth in SEQ ID NO: 19;
      c. a strain comprising a genomic marker comprising the nucleic acid sequence set forth in SEQ ID NOs:72; and any combination thereof.
2. The isolated bacterial strain or a functional homologue thereof according to aspect 1, wherein strain LAV49762, strain LAV46348, strain LAV43122, strain LAV48853, strain LAV60063, strain LAV60064, and strain LAV60072 each is of the genus *Pseudomonas.*
3. The isolated bacterial strain or a functional homologue thereof according to aspect 1, wherein strain LAV34085 is of the genus *Serratia.*
4. The isolated bacterial strain or a functional homologue thereof according to aspect 1, wherein strain LAV43723 is of the genus *Streptomyces.*
5. The isolated bacterial strain or a functional homologue thereof according to aspect 1, wherein strain LAV49623 and strain LAV59924 each is of the genus *Pantoea.*
6. The isolated bacterial strain or a functional homologue thereof according to aspect 1, wherein strain LAV49648 and strain LAV60070 each is of the genus *Enterobacter.*
7. The isolated bacterial strain or a functional homologue thereof according to aspect 1, wherein strain LAV54823 is of the genus *Erwinia.*
8. The isolated bacterial strain or a functional homologue thereof according to aspect 1, wherein strain LAV60069 and strain LAV61190 each is of the genus *Bacillus.*
9. The isolated bacterial strain or a functional homologue thereof according to aspect 1, wherein strain LAV60067 is of the genus *Gluconobacter.*
10. The isolated bacterial strain according to aspects 1 or 2, wherein:
   the functional homolog of strain LAV49762 comprises a 16S-rRNA sequence at least 97% identical to SEQ ID NO: 10; at least one genomic marker having at least 95% local identity to a nucleic acid sequence set forth in any one of SEQ ID NOs:53-57 over 90% coverage; or a combination thereof;
   the functional homolog of strain LAV46348 comprises a 16S-rRNA sequence at least 98% identical to SEQ ID NO:5; at least one genomic marker having at least 95% local identity to a nucleic acid sequence set forth in any one of SEQ ID NOs:33-37 over 90% coverage; or a combination thereof;
   the functional homolog of strain LAV43122 comprises a 16S-rRNA sequence having the nucleic acid sequence set forth in any one of SEQ ID NO:2 and
   SEQ ID NO:3 and at least one genomic marker having at least 95% local identity to a nucleic acid sequence set forth in any one of SEQ ID NOs:26-27 over 90% coverage;
   the functional homolog of bacterial strain LAV48853 comprises a 16S-rRNA sequence at least 99% identical to SEQ ID NO:6; at least one genomic marker having at least 95% local identity to a nucleic acid sequence set forth in any one of SEQ ID NOs:38-42 over 90% coverage; or a combination thereof;
   the functional homolog of bacterial strain LAV60063 comprises a 16S-rRNA sequence at least 99% identical to SEQ ID NO: 13; at least one genomic marker having at least 95% local identity to a nucleic acid sequence set forth in any one of SEQ ID NOs:58-59 over 90% coverage; or a combination thereof;
   the functional homolog of bacterial strain LAV60064 comprises a 16S-rRNA sequence at least 97% identical to SEQ ID NO: 14; at least one genomic marker having at least 95% local identity to a nucleic acid sequence set forth in any one of SEQ ID NOs:60-61 over 90% coverage; or a combination thereof; and/or
   the functional homolog of bacterial strain LAV60072 comprises a 16S-rRNA sequence at least 97% identical to SEQ ID NO: 19; a genomic marker having at least 95% local identity to the nucleic acid sequence set forth in SEQ ID NO:72 over 90% coverage; or a combination thereof.
11. The isolated bacterial strain according to aspects 1 or 3, wherein the functional homolog of strain LAV34085 comprises a 16S-rRNA sequence at least 97% identical to SEQ ID NO:1; at least one genomic marker having at least 95% local identity to a nucleic acid sequence set forth in any one of SEQ ID NOs:21- 25 over 90% coverage; or a combination thereof.
12. The isolated bacterial strain according to aspects 1 or 4, wherein the functional homolog of strain LAV43723 comprises a 16S-rRNA sequence at least 97% identical to SEQ ID NO:4; at least one genomic marker having at least 95% local identity to a nucleic acid sequence set forth in any one of SEQ ID NOs:28- 32 over 90% coverage; or a combination thereof.
13. The isolated bacterial strain according to aspects 1 or 5, wherein:
   the functional homolog of strain LAV49623 comprises a 16S-rRNA sequence at least 97% identical to SEQ ID NO:7; at least one genomic marker having at least 95% local identity to a nucleic acid sequence set forth in any one of SEQ ID NOs:43-47over 90% coverage; or a combination thereof; and/or
   the functional homolog of bacterial strain LAV59924 comprises a 16S-rRNA sequence at least 97% identical to SEQ ID NO: 12.
14. The isolated bacterial strain according to aspects 1 or 6, wherein: the functional homolog of bacterial strain LAV49648 comprises a 16S-rRNA sequence having the nucleic acid sequence set forth in any one of SEQ ID NO: 8 and SEQ ID NO:9 and at least one genomic marker having at least 95% local identity to a nucleic acid sequence set forth in any one of SEQ ID NOs:48-52 over 90% coverage; or a combination thereof.
15. The isolated bacterial strain according to aspects 1 or 8, wherein:
   the functional homolog of bacterial strain LAV60069 comprises a 16S-rRNA sequence at least 97% identical to SEQ ID NO: 16; at least one genomic marker having at least 95% local identity to a nucleic acid sequence set forth in any one of SEQ ID NOs:67-71 over 90% coverage; or a combination thereof; and/or
   the functional homolog of bacterial strain LAV61190 comprises a 16S-rRNA sequence at least 97% identical to SEQ ID NO:20.
16. The isolated bacterial strain according to aspects 1 or 9, wherein:
   the functional homolog of bacterial strain LAV60067 comprises a 16S-rRNA sequence at least 97% identical to SEQ ID NO: 15; at least one genomic marker having at least 95% local identity to a nucleic acid sequence set forth in any one of SEQ ID NOs:62-66 over 90% coverage; or a combination thereof.
17. The isolated bacterial strain or functional homolog thereof according to any one of aspects 1-16, wherein said strain or functional homolog is characterized by a capability to inhibit the growth and/or the development and/or the activity of at least one plant pathogenic fungus and/or oomycete.
18. The isolated bacterial strain or functional homolog thereof according to any one of aspects 1-17, wherein said bacterial strain or functional homolog is effective in protecting a plant from at least one pathogenic fungus and/or oomycete.
19. A bacterial preparation comprising a plurality of bacteria of at least one isolated bacterial strain or functional homolog thereof according to any one of aspects 1-18.
20. The bacterial preparation according to aspect 19, wherein said bacterial preparation comprises the plurality of bacteria of at least one bacterial strain or functional homolog thereof at a concentration which exceeds that found in nature.
21. The bacterial preparation according to any one of aspects 19-20, said bacterial preparation comprising the plurality of bacteria of at least one bacterial strain or functional homolog thereof is in a form selected from the group consisting of viable form and non-viable form.
22. The bacterial preparation according to aspect 21, comprising the plurality of bacteria of at least one bacterial strain or functional homolog thereof in a non- viable form, wherein said bacterial preparation has been subjected to a temperature of from about 100°C to about 130°C under pressure of at least 15psi for at least 10 min.
23. The bacterial preparation according to any one of aspects 19-22, wherein said bacterial preparation is substantially stable for at least 180 days at a temperature range of from about 4°C to about 37°C.
24. A lysate of at least one bacterial cell of the at least one isolated bacterial strain or functional homolog thereof according to any one of aspects 1-18.
25. The lysate according to aspect 24, said lysate being selected from the group consisting of the at least one cell whole lysate and lysate comprising soluble fraction of the at least one cell.
26. An extract of at least one bacterial cell of the at least one isolated bacterial strain or functional homolog thereof according to any one of aspects 1-18.
27. An agricultural composition comprising at least one isolated bacterial strain or functional homolog thereof according to any one of aspects 1-18, a bacterial preparation according to any one of aspects 19-23, a lysate according to any one of aspects 24-25 or an extract according to aspect 26, further comprising agriculturally acceptable diluents or carriers.
28. The agricultural composition of aspect 27, wherein the carrier is a plant seed.
29. The agricultural composition according to any one of aspects 27-28, wherein said agricultural composition is a plant protection product effective in preventing or treating at least one plant disease caused by a pathogenic fungus and/or oomycete.
30. The agricultural composition according to any one of aspects 27-29, wherein said agricultural composition is substantially stable for at least 180 days at a temperature range of from about 4°C to about 37°C.
31. A method for enhancing and/or conferring resistance to a plant or a part thereof toward a disease caused by a phytopathogenic fungus and/or oomycete, comprising contacting the plant or part thereof with at least one bacterial strain or a functional homolog thereof according to any one of aspects 1-18, a bacterial preparation according to any one of aspects 19-23, a lysate according to any one of aspects 24-25, an extract of aspect 26 or an agricultural composition comprising same according to any one of aspects 27-30.
32. The method according to aspect 31, said method further comprising identifying a plant to be susceptible to the at least one disease caused by the phytopathogenic fungus and/or oomycete before contacting the plant or a part thereof with the at least one bacterial strain or functional homolog thereof.
33. A method for preventing or treating a plant disease caused by a phytopathogenic fungus and/or oomycete, comprising contacting a plant or a part thereof with at least one bacterial strain or a functional homolog thereof according to any one of aspects 1-18, a bacterial preparation according to any one of aspects 19-23, a lysate according to any one of aspects 24-25, an extract of aspect 26 or an agricultural composition comprising same according to any one of aspects 27- 30.
34. The method according to aspect 33, said method further comprising identifying symptoms of the disease within a plant before contacting the plant or a part thereof with the at least one bacterial strain or functional homolog thereof.
35. The method according to any one of aspects 31-34, said method comprising contacting the plant or part thereof with at least one of:
   a. a functional homolog of bacterial strain LAV43122, the functional homolog comprising a 16S-rRNA sequence at least 97% identical to a nucleic acid sequence selected from the group consisting of SEQ ID NO:2 and SEQ ID NO:3; a genomic marker having at least 95% local identity to a nucleic acid sequence set forth in any one of SEQ ID NOs:26-27 over 90% coverage; or a combination thereof;
   b. a functional homolog of bacterial strain LAV49648, the functional homolog comprising a 16S-rRNA sequence at least 97% identical to a nucleic acid sequence selected from the group consisting of SEQ ID NO: 8 and SEQ ID NO:9; a genomic marker having at least 95% local identity to a nucleic acid sequence set forth in any one of SEQ ID NOs:48-52 over 90% coverage; or a combination thereof;
   c. a functional homolog of bacterial strain LAV54823, the functional homolog comprises a 16S-rRNA sequence at least 97% identical to a nucleic acid sequence set forth in SEQ ID NO: 11; and
   d. a functional homolog of bacterial strain LAV60070, the functional homolog comprising a 16S-rRNA sequence at least 97% identical to SEQ ID NO: 17; a 16S-rRNA sequence having the nucleic acid sequence set forth by SEQ ID NO: 18; or a combination thereof;
   a bacterial preparation of same, a lysate thereof, and extract thereof or an agricultural composition comprising same.
36. The method according to any one of aspects 31-35, wherein the plant part is selected from the group consisting of a seed, a root, a shoot, a leaf, a branch, a flower, a fruit and any combination thereof.
37. The method according to any one of aspects 31-36, wherein the plant or part thereof is contacted with the bacterial preparation, lysate, extract or agricultural composition comprising same by a method selected from the group consisting of infiltration, immersion, dipping, incubation, spraying, dusting and any combination thereof.
38. The method according to any one of aspects 31-36, wherein the plant or part thereof is contacted with the bacterial preparation, lysate, extract or agricultural composition comprising same indirectly, via administration of said bacterial preparation, lysate, extract or agricultural composition comprising same to the plant growth medium.
39. The method according to aspect 36, wherein the plant part is a seed and the bacterial preparation, lysate, extract or agricultural composition comprising same is applied via seed coating.
40. The method according to any one of aspects 31-39, wherein the bacterial preparation or agricultural composition comprises from about 10⁵ to about 10⁸ CFU/ml.
41. The method according to any one of aspects 31-40, wherein the phy topathogenic fungus is of a genus selected from the group consisting of, *Fusarium, Botrytis, Erysiphe, Aspergillus* and *Rhizopus.*
42. The method according to aspect 41, wherein the fungus is selected from the group consisting of *Fusarium verticillioid.es, Botrytis cinerea, Fusarium graminearum, Fusarium oxysporum, Erysiphe necator, Aspergillus niger,* and *Rhizopus stolonifera.*
43. The method according to any one of aspects 31-40, wherein the phytopathogenic oomycete is of a genus selected from the group consisting of *Plasmopara, Phytophthora, Pythium,* and *Pseudoperonospora.*
44. The method according to aspect 43, wherein the oomycete is selected from the group consisting of *Plasmopara viticola, Phytophthora capsica,* and *Pseudoperonospora cubensis.*
45. The method according to aspect 41, wherein the pathogenic fungus is selected from the group consisting of *Fusarium graminearum* and *Fusarium verticilloides,* and the bacterial strain is selected from the group consisting of LAV43122, LAV46348, LAV49623, LAV60067, LAV61190, functional homologs thereof and any combination thereof.
46. The method according to aspect 43, wherein the pathogenic oomycete is *Phytophthora capsici* and the bacterial strain is selected from the group consisting of LAV43122, LAV46348, LAV48853, LAV59924, LAV60067, LAV60064, LAV60072, LAV61190, functional homologs thereof, and any combination thereof.
47. The method according to aspect 43, wherein the pathogenic oomycete is *Plasmopara viticola,* and the bacterial strain is selected from the group consisting of LAV43723, LAV61190, functional homologs thereof and any combination thereof.
48. The method according to aspect 47, wherein the plant contacted with the bacterial strain is a grapevine.
49. The method according to aspect 41, wherein the pathogenic fungus is *Erysiphe necator,* and the bacterial strain is selected from the group consisting of LAV49648, LAV43122, LAV54823, LAV48853, LAV60062, LAV60063, LAV60069, LAV60070, LAV60072, functional homologs thereof, and any combination thereof.
50. The method according to aspect 49, wherein the plant contacted with the bacterial strain is a grapevine.
51. The method according to aspect 41, wherein the pathogenic fungus is *Botrytis cinerea,* and the bacterial strain is selected from the group consisting of LAV43122, LAV54823, LAV49762, LAV49648, LAV49623, LAV34085, LAV46348, LAV59924, LAV60063, LAV60064, LAV60067, LAV60069, LAV60070, LAV60072, LAV60096, functional homologs thereof and any combination thereof.
52. The method according to aspect 51, wherein the plant contacted with the bacterial strain is selected from the group consisting of a tomato plant and a grapevine.
53. The method according to aspect 41, wherein the pathogenic fungus is selected from the group consisting of *Fusarium oxysporum* and *Fusarium graminearum,* and the bacterial strain is selected from the group consisting of LAV43122, LAV46348, LAV49623, LAV60067, LAV61190, functional homologs thereof and any combination thereof.
54. The method according to aspect 53, wherein the plant contacted with the bacterial strain is selected from the group consisting of a tomato plant and a maize plant.
55. The method according to aspect 41, wherein the pathogenic fungus is *Aspergillus niger,* and the bacterial strain is selected from the group consisting of LAV54823, LAV49762, functional homologs thereof and any combination thereof.
56. The method according to aspect 55, wherein the plant contacted with the bacterial strain is a grapevine.

## Claims

1. An isolated bacterial strain, wherein the isolated bacterial strain is strain LAV54823 deposited under Accession Number 43433 at NCIMB.

2. A bacterial preparation comprising a plurality of bacteria of the isolated bacterial strain according to claim 1.

3. The bacterial preparation of claim 2, wherein the bacterial preparation comprises the plurality of bacteria of the bacterial strain at a concentration which exceeds that found in nature;
and/or wherein said bacterial preparation comprises the plurality of bacteria of the bacterial strain in a form selected from the group consisting of viable form and non-viable form;
preferably wherein said bacterial preparation comprises the plurality of bacteria of the bacterial strain in a non-viable form, wherein said bacterial preparation has been subjected to a temperature of from about 100°C to about 130°C under pressure of at least 15psi for at least 10 min.

4. A lysate of at least one bacterial cell of the isolated bacterial strain according to claim 1.

5. The lysate of claim 4, wherein the lysate is selected from the group consisting of a whole lysate of the at least one cell and a lysate comprising a soluble fraction of the at least one cell.

6. An extract of at least one bacterial cell of the isolated bacterial strain according to claim 1.

7. An agricultural composition:
(i) comprising the isolated bacterial strain according to claim 1, the bacterial preparation according to claim 2 or 3, the lysate according to claim 4 or 5, or the extract according to claim 6, further comprising agriculturally acceptable diluents or carriers; or
(ii) comprising the isolated bacterial strain according to claim 1, the bacterial preparation according to claim 2 or 3, the lysate according to claim 4 or 5, or the extract according to claim 6:
(a) formulated as an emulsion, wettable powder, granule, gel, pellet, microencapsulated particle, aqueous suspension, or aqueous flowable; and/or
(b) further comprising an agriculturally effective amount of an active agent selected from the group consisting of: a fertilizer, an acaricide, a bactericide, a fungicide, an insecticide, a microbicide, a nematicide, a pesticide, a plant growth regulator, a rodenticide, and a nutrient.

8. The agricultural composition of claim 7, wherein the carrier is a plant seed.

9. A method for enhancing and/or conferring resistance to a plant or a part thereof toward a disease caused by a phytopathogenic fungus, comprising contacting the plant or part thereof with the bacterial strain according to claim 1, the bacterial preparation according to claim 2 or 3, the lysate according to claim 4 or 5, the extract according to claim 6, or the agricultural composition according to claim 7 or 8.

10. The method of claim 9, wherein said method further comprises identifying a plant to be susceptible to the at least one disease caused by the phytopathogenic fungus before contacting the plant or a part thereof with the bacterial strain.

11. A method for preventing or treating a plant disease caused by a phytopathogenic fungus, comprising contacting a plant or a part thereof with the bacterial strain according to claim 1, the bacterial preparation according to claim 2 or 3, the lysate according to claim 4 or 5, the extract according to claim 6, or the agricultural composition comprising the same according claim 7 or 8.

12. The method of claim 11, wherein said method further comprises identifying symptoms of the disease within a plant before contacting the plant or a part thereof with the bacterial strain.

13. The method according to any one of claims 9-12, wherein the plant part is selected from the group consisting of a seed, a root, a shoot, a leaf, a branch, a flower, a fruit and any combination thereof;
and/or wherein the plant or part thereof is contacted with the bacterial preparation, lysate, extract or agricultural composition comprising the same:
(i) by a method selected from the group consisting of infiltration, immersion, dipping, incubation, spraying, dusting and any combination thereof; or
(ii) indirectly, via administration of said bacterial preparation, lysate, extract or agricultural composition comprising same to the plant growth medium;
preferably wherein the plant part is a seed and the bacterial preparation, lysate, extract or agricultural composition comprising same is applied via seed coating;
and/or wherein the bacterial preparation or agricultural composition comprises from about 10⁵ to about 10⁸ CFU/ml.

14. The method according to any one of claims 9-13, wherein the phytopathogenic fungus is of a genus selected from the group consisting of *Botrytis* and *Aspergillus,*
preferably wherein the fungus is selected from the group consisting of *Botrytis cinerea* and *Aspergillus niger.*

15. The method according to claim 14, wherein:
(i) the pathogenic fungus is *Botrytis cinerea,* preferably wherein the plant contacted with the bacterial strain is selected from the group consisting of a tomato plant and a grapevine; or
(ii) the pathogenic fungus is *Aspergillus niger*; preferably wherein the plant contacted with the bacterial strain is a grapevine.
